# EUROPEAN PATENT SPECIFICATION

(11) **EP 1 664 110 B1**
(45) Date of publication and mention of the grant of the patent: **20.08.2008**
(21) Application number: 04739031.5
(22) Date of filing: 12.08.2004
(51) Int. Cl.: C07K 14/705, C07K 1/113, C12N 15/10, B03C 1/00

(54) **A CONTINUOUS PROCESS FOR THE ASSEMBLY OF MACROMOLECULAR SUBSTANCES AND THE SUBSEQUENT CAPTURE AND ISOLATION OF A MACROMOLECULAR ASSEMBLY, AND A SYSTEM SUITABLE FOR THE PROCESS**
KONTINUIERLICHES VERFAHREN ZUR ANORDNUNG VON MAKROMOLEKULÄREN SUBSTANZEN UND DIE ANSCHLIESSENDE AUFNAHME UND ISOLIERUNG EINER MAKROMOLEKULÄREN ANORDNUNG, SOWIE EIN FÜR DIESES VERFAHREN GEEIGNETES SYSTEM
PROCEDE CONTINU D'ASSEMBLAGE DE SUBSTANCES MACROMOLECULAIRES ET CAPTURE ET ISOLATION ULTERIEURES D'UN ENSEMBLE MACROMOLECULAIRE, ET SYSTEME APPROPRIE POUR CE PROCEDE

(30) Priority: 26.08.2003 DK 200301217; 26.08.2003 US 497574 P
(43) Date of publication of application: 07.06.2006
(73) Proprietor: Danmarks Tekniske Universitet, 2800 Lyngby (DK); Kobenhavns Universitet, 1017 Copenhagen K (DK)
(72) Inventor: FERRE, Henrik, DK-2720 Vanlöse (DK); HANSEN, Dennis, B., DK-1620 Copenhagen V (DK); BUUS, Sören, DK-2830 Virum (DK); HOBLEY, Timothy, J., DK-2300 Copenhagen S (DK); THOMAS, Owen, R., T., Birmingham B31 2LB (GB)
(74) Representative: Inspicos A/S
(86) International application number: PCT/DK2004/000534
(87) International publication number: WO 2005/019263

(56) References cited:
- WO-A-00/70040
- WO-A-01/52612
- WO-A-02/44327
- WO-A-99/28748
- WO-A-02/057296
- US-A1- 2003 032 028
- HUBBUCH JJ ET AL.: "High gradient magnetic separation versus expanded bed adsorption: a first principle comparison" BIOSEPARATION, vol. 10, no. 1-3, 2001, pages 99-112, XP008037365 cited in the application
- MANNEN T ET AL.: "Expanded-Bed Protein Refolding Using a Solid-Phase Artificial Chaperone" JOURNAL OF BIOSCIENCE AND BIOENGINEERING, vol. 91, no. 4, April 2001 (2001-04), pages 403-408, XP002303004
- STEMPFER G ET AL.: "Improved Refolding of an Immobilized Fusion Protein" NATURE BIOTECHNOLOGY, vol. 14, no. 3, March 1996 (1996-03), pages 329-334, XP001182925
- SHIMIZU H ET AL.: "Renaturation of Reduced Ribonuclease A with a Microsphere-Induced Refolding System" BIOTECHNOLOGY PROGRESS, vol. 16, no. 2, 23 March 2000 (2000-03-23), pages 248-253, XP008037816

## Description

### FIELD OF THE INVENTION

The present invention relates to the field of macromolecular assembly and capture, e.g. to the process of refolding of proteins, hybridization of nucleic acid, nucleic acid analogues, and protein-nucleic acid chimera, and aggregation of carbohydrates. The present invention provides a continuous process for assembly of macromolecular substances and capture of a macromolecular assembly of one or more macromolecular substances. The present invention also provides a system suitable for the process.

### BACKGROUND OF THE INVENTION

Assembly of macromolecular substances plays an important role in biotechnology and within nanotechnology. Modem biotechnology promises to supply an unlimited amount of scarce, high-value proteins. Expression of recombinant proteins in bacteria is widely used due to the ease of genetic manipulation, and the reasonably simple growth and induction methodologies, leading to high yield and purity of the desired product. However, high levels of expression frequently lead to deposition of aggregated inactive proteins in the form of insoluble inclusion bodies within the cytosol of the bacterial cell. To regain the biological activity of the protein of interest, solubilisation of the inclusion bodies by extraction into denaturing agents is required followed by some kind of *in vitro* assembly reaction. Thus, the high yields of bacterial expression do not readily translate into high yields of functional protein.

Extraction of inclusion body proteins into a denaturing agent, such as urea, results in a soluble protein structure, which in an unfolded state devoid of biological activity. For a monomeric unfolded protein, the assembly process can be spontaneously initiated simply by removing the denaturant, since the unfolded state possesses the required information to guide the molecule towards the correct 3-dimensional structure. This kind of assembly process is most often referred to as protein refolding in the scientific literature. However, in the case of more complex macromolecular structures composed of two or more separate protein domains or other components such as carbohydrates, nucleic acids, nucleic acid analogues, and protein-nucleic acid chimera, the process becomes more complicated as these structures must be added together either in an unfolded/unassembled and/or folded/assembled state in a defined ratio to generate the correct final form.

Adding to the complexity of the above-mentioned assembly processes is the fact that these reactions are associated with the formation of soluble and more importantly insoluble by-produds. Such by-products are generated due to unfavourable intra- and/or inter-molecular interactions within or between the components that constitute the final structure of the macromolecule. Intramolecular interactions (interactions within a molecule) are concentration independent, whereas intermolecular interactions (interactions between neighbouring molecules) are highly concentration dependent. For a single protein assembly reaction (i.e. assembly of a single protein domain from the unfolded state) the situation can therefore be improved by lowering the concentration of the reactants to minimize the likelihood of interaction between neighbouring molecules. However, dilution leads to the problem of handling large volumes of solutions and as the formation of Insoluble by-products cannot be completely avoided, the net outcome of an assembly process is likely to be a situation in which large volumes of unclarified suspensions must be dealt with.

Assembly reactions of the nature described above are commonly conducted in a batch-wise manner, where the protein components are added together in a single reactor and stirred for a period of time to allow the reaction to proceed to completion.

In such processes, only the initial assembly conditions can be defined and controlled as the ratio and concentration of protein components will change with time as more and more correct or incorrect structures form. Technically, mixing of large volumes in a batch reactor is a demanding task and even under optimal conditions, it is difficult to obtain a homogenous and well-defined system. Lack of control affects the reproducibility of the process, which is a crucial parameter in the governmental approval of processes for production of pharmaceutical products. Furthermore, batch reactions are associated with much longer hold-up times than continuous processes, which increase the risk of contamination and inadvertent modifications of the product, such as proteolytic degradation.

Following the batch assembly process, the product must be concentrated and purified, which is conventionally done by packed bed chromatography. However, as the assembly reaction usually generates particulate by-products, the solution must be treated by centrifugation and/or filtration prior to the packed bed chromatography step, as the presence of insoluble substances will otherwise cause fouling of the columns. The higher the number of unit operations the product must go through, the greater the reductions in yield and increase in production costs. Alternatively, chromatographic beads or magnetic particles can be added directly to the batch mixture to 'fish' out the protein of interest. However, in a batch adsorption process, equilibrium between the amounts of bound and unbound molecules is formed and in order to remove all of the product, loaded support must be collected, followed by adding fresh and this sequence must be repeated several times.

It should be mentioned that a number of scientific fields other than those described above exist in which the use of efficient macromolecular assembly processes could be of great value, including but not limited to gene therapy for assembly of vaccine carriers.

Use of High Gradient Magnetic Separation (HGMS) for purification of biological entities from different sources, including porcine pancreatin (Hubbuch et al., 2002), whey, jack bean extract and solubilised inclusion bodies (Heebøll-Nielsen, 2002) is known. Further, HGMS and Expanded Bed Adsorption as separation methods have been compared (Hubbuch et al., 2001).

US 5,123,901 discloses a method for continuous-flow removal of pathogenic agents such as cells or viruses from whole blood using functionalized magnetic beads. This is achieved by allowing a stream of magnetic beads, functionalized to specifically bind a pre-selected target molecule, to be introduced into a stream of continuously flowing blood from a patient. The two streams are mixed by passage through a suitable mixing device, such as a coil, to promote contacting and binding of the target molecules to the magnetic beads. Pathogen-loaded magnetic beads are subsequently retained in the tubing by flowing through a magnetic separator and the purified stream of blood is re-directed back into the patient. It is mentioned in the description that the mixing coil could include re-circulating shunts and the like to ensure proper contact between particles and fluid.

However, US 5,123,901 does not address the problem of concurrent assembly of macromolecular substances, and does not address the problem of isolating such macromolecular substances.

WO 02/057296 discloses a method for continuous refolding of proteins in which the conditions for folding can be specifically set and maintained throughout the reaction and directly coupled to a separation process. Constant folding conditions are obtained by continuous dilution of a denatured protein suspension, comprising the protein in an unfolded state, in a mixing device. The outlet of the mixer can be directly connected to a separation process in order to permit the capture of the refolded protein after the folding event. A preferred embodiment of the invention uses Expanded Bed Adsorption (EBA) as the separation step. The possibility of using functionalized magnetic particles and high gradient magnetic separation for the separation is not mentioned anywhere in WO 02/057296.

Therefore, there is a need to provide methods in which the assembly process can be performed under continuous and precisely defined conditions for improved throughput and reproducibility. Furthermore, these methods should integrate separation techniques that can handle non-clear suspensions in order to bypass the need for centrifugation and filtration. The present invention achieves these goals.

### BRIEF DESCRIPTION OF THE INVENTION

One aspect of the present invention relates to a continuous process for obtaining a preparation of a macromolecular assembly of one or more macromolecular substances including at least one substance selected from the group consisting of proteins, carbohydrates, nucleic acids, nucleic acid analogues, and protein-nucleic acid chimera, said process comprising the steps of:
a) (i) providing one or more fluid compositions each comprising at least one of said one or more macromolecular substances, said one or more macromolecular substances being in a predominantly unassembled form, (ii) providing a dispersion comprising coated, essentially non-porous magnetic particles, and (iii) optionally providing one or more additives;
b) simultaneously or sequentially combining said one or more fluid compositions, the dispersion of magnetic particles, and said one or more optional additives so as to provide a continuous stream comprising said one or more macromolecular substances, the magnetic particles and any optional additives;
c) passing said continuous stream through a first mixing device so as to form magnetic complexes each comprising a magnetic particle and a plurality of macromolecular assembly species;
d) passing the continuous stream through a first capture compartment of a magnetic separator so as to capture said magnetic complexes; and
e) separating said magnetic complexes from said continuous stream, and isolating said macromolecular assembly species from said magnetic particles so as to obtain the preparation of a macromolecular assembly of said one or more macromolecular substances.

Another aspect of the present invention relates to a system useful for the continuous assembly and capture of macromolecular substances, the system comprising: a plurality of containers including at least one first container containing a dispersion of magnetic particles (8), at least one second container containing a liquid composition comprising one or more macromolecular substances (1), and optionally at least one third container (2) containing one or more additives; at least one pump(s) (3,4,7) for causing the content of each of said containers (1,2,8) to be fed to a first mixing device (10,11); a conduit for providing passage from said first mixing device (10,11) to the capture compartment (16) of a magnetic separator (15), said magnetic separator (15) having at least two operational modes, a first of the at least two operational modes providing a magnetic field to the capture compartment (16) thereby rendering the capture compartment (16) capable of capturing the magnetic particles (8), and a second of the at least two operational modes providing an inadequate magnetic field to the capture compartment (16) thereby rendering the capture compartment (16) substantially incapable of capturing the magnetic particles (8).

### BRIEF DESCRIPTION OF THE DRAWINGS

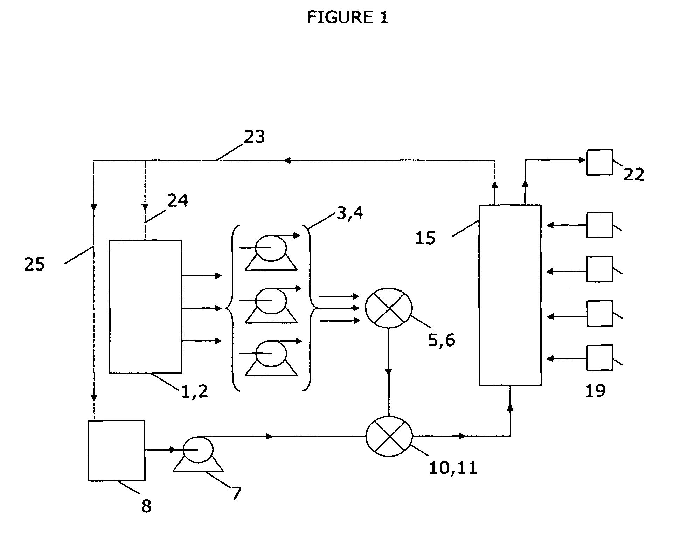

Figure 1. Schematic illustration of an embodiment of the system of the present invention.

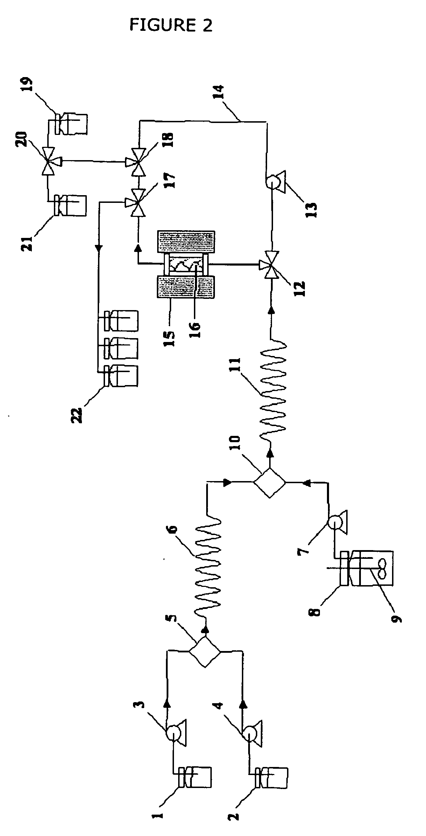

Figure 2. Schematic illustration of an embodiment of the system according to the invention. The system was used for continuous refolding and adsorption of HAT-hβ2m on magnetic chelate adsorbents combined with separation and product elution in a high gradient magnetic separator (Example 1, etc.).

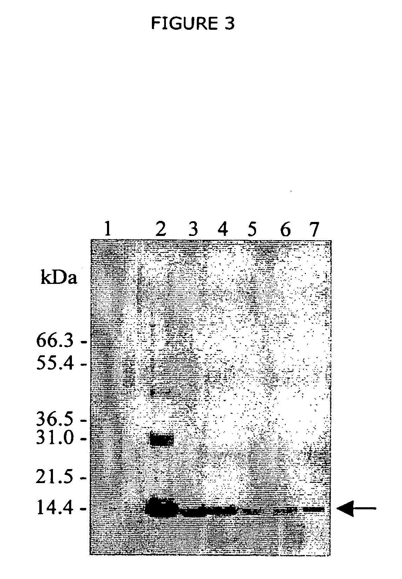

Figure 3. Non-reducing SDS-PAGE analysis of collected fractions during elution of bound HAT-hp2m from Cu²⁺-charged magnetic chelators in a high gradient magnetic separator. Lanes: 1: Protein standards, 2: Feedstock B, 3-7: Successive elution with 500 mM imidazole in 500 mM NaCl, 20 mM Tris, pH 8. Molecular weights of standard proteins are shown on the figure and the position of the monomeric HAT-hβ2M molecule is indicated with an arrow.

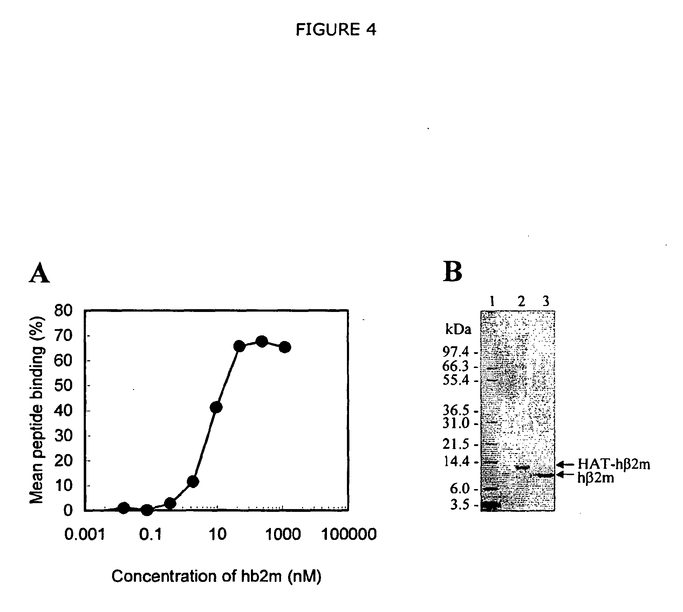

Figure 4. Investigation of biological activity of the hβ2m produced. (A) Dose response curve for hβ2m. Folding buffer containing graded concentrations of hβ2m and a fixed amount of radiolabeled peptide were prepared and used to dilute a fixed amount of denatured MHC-I heavy chain. The folding reaction leading to complex formation and peptide binding was allowed to proceed for 24 h at 18 °C before separation of bound and unbound peptides by spin-column chromatography as described in Materials and methods. (B) SDS-PAGE analysis of HAT-hp2m before and after cleavage with Factor Xa. Lanes: 1: Protein standards, 2: Before cleavage (HAT-hβ2M), 3: After cleavage (hβ2M). Equal amounts (5 µg) of protein were applied to the gel. Molecular weights of standard proteins are shown on the figure and the positions of HAT-hβ2M and hp2M are indicated with arrows.

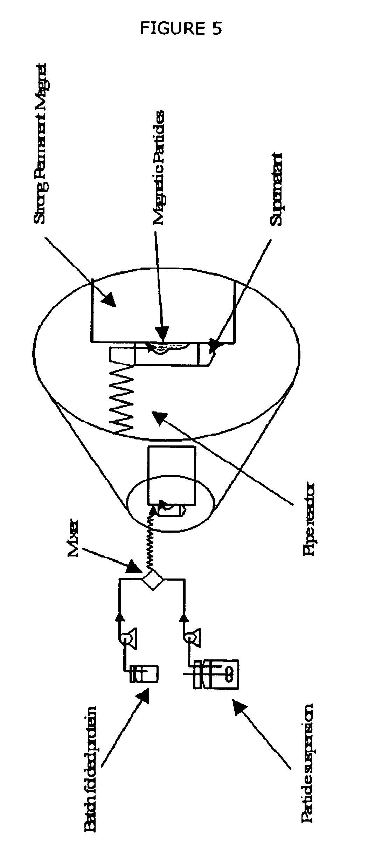

Figure 5. Schematic illustration of an embodiment of the system according to the invention. The system was used for determining the conditions for continuous binding of folded HAT-hβ2m to magnetic supports. Part of the diagram featuring the pipe reactor and the magnetic separator has been enlarged to aid understanding.

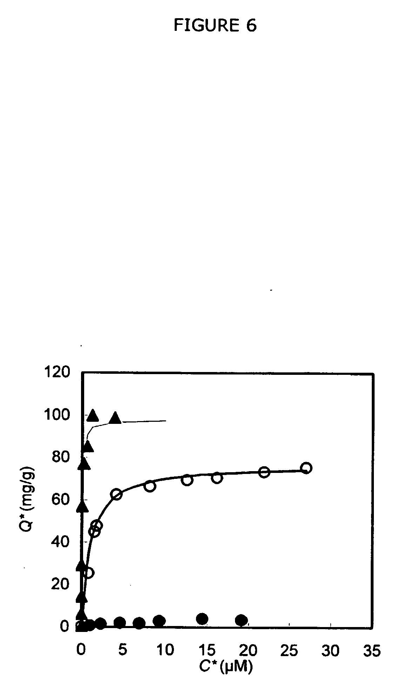

Figure 6. Equilibrium binding isotherms for adsorption of folded HAT-hβ2m (triangles) and BSA (circles) to uncharged (•) and Cu²⁺-charged (o, ▲) IDA-linked magnetic adsorbents. Solid lines through the data represent the fit of Langmuir isotherms with the parameters summarised in Table 3.

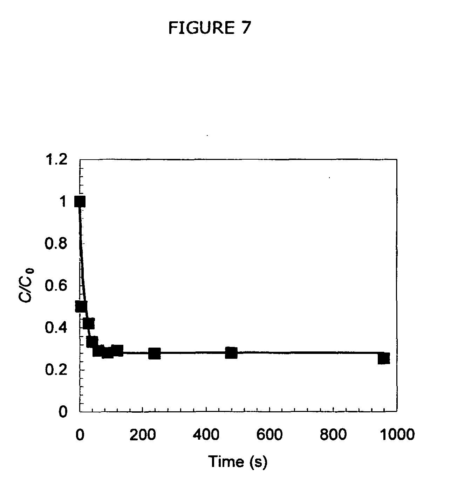

Figure 7. Binding kinetics for adsorption of folded HAT-hβ2M to Cu²⁺-charged IDA-linked magnetic supports. The solid line indicates the empirical solution (Sharma and Agarwal (2002)) to the mass-transfer model described by Chase (1984).

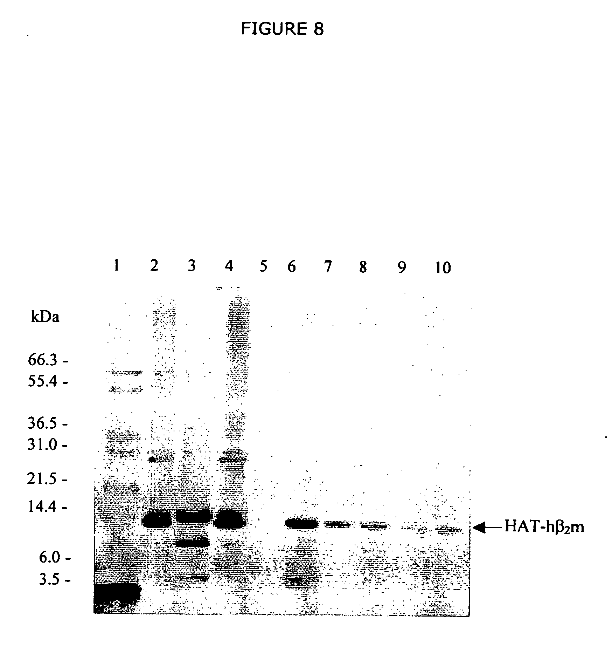

Figure 8. SDS-PAGE analysis of collected fractions from batch elution of HAT-hβ2M from Cu²⁺-charged IDA-linked supports. Lanes: 1: Protein standards, 2: Non-reduced Feedstock A, 3: Reduced Feedstock A, 4: Batch folded suspension, 5: Wash, 6-10: successive elutions with 500 mM of imidazole, 20 mM Tris-HCl, pH 8. Molecular weights of standard proteins are shown in the figure and the position of the monomeric HAT-hβ2M molecule is indicated by an arrow.

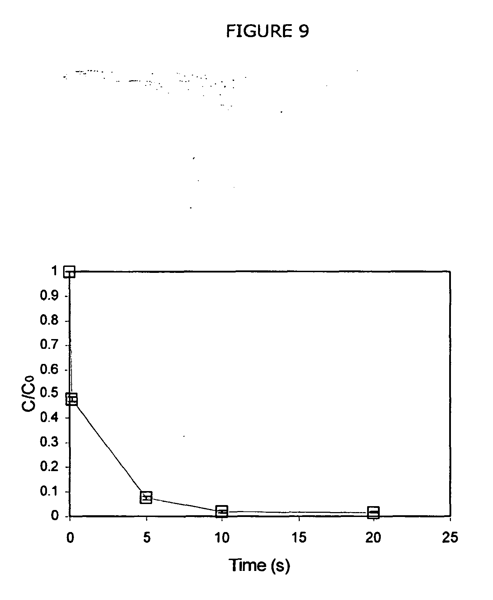

Figure 9. Continuous adsorption of batch folded HAT-hp2m onto Cu²⁺-charged IDA-linked magnetic supports under turbulent flow conditions. The ratio of unbound (C) and bound (C₀) product is plotted against the mixing time. Particle concentration during adsorption was 3.0 mg/ml and the total amount of protein was 100 µg/ml. Batch folding was conducted at a protein concentration of 200 µg/ml and the final urea concentration was 266 mM.

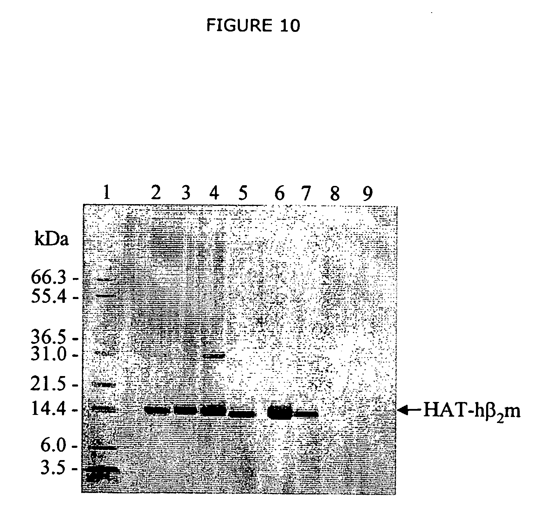

Figure 10. SDS-PAGE analysis of elution fractions collected after continuous adsorption under turbulent conditions of batch folded HAT-hβ2M to Cu²⁺ -charged IDA-linked supports. Lanes: 1: Protein standards 2: Non-reduced Feedstock B, 3: Reduced Feedstock B, 4: Batch folded suspension, 5-9: successive elutions with 500 mM imidazole, 500 mM NaCl, 20 mM Tris-HCl, pH 8. Molecular weights of standard proteins are shown in the figure and the position of the monomeric HAT-hβ2M molecule is indicated by an arrow.

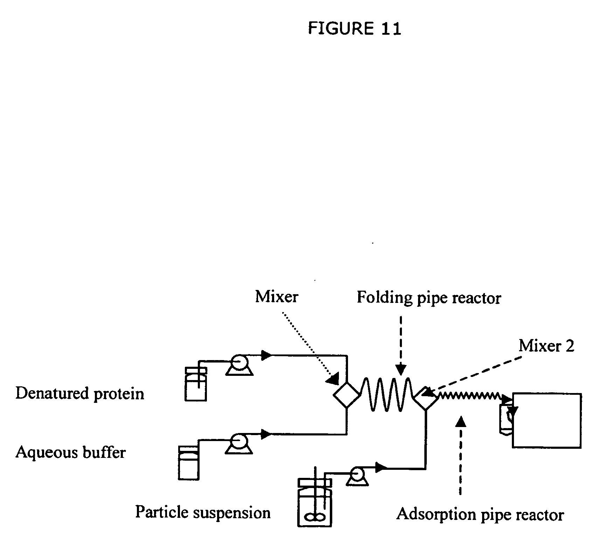

Figure 11. Schematic diagram of system used to examine operating conditions for continuous refolding and adsorption of HAT-hβ2m onto magnetic metal chelate adsorbents.

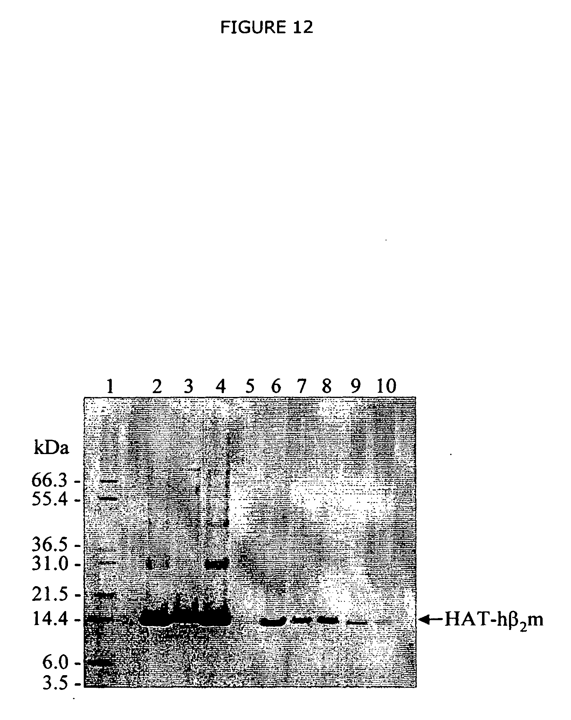

Figure 12. SDS-PAGE analysis of fractions collected from batch elution of HAT-hβ2M after continuous folding and capture onto Cu²⁺-charged IDA-linked particles. Lanes: 1: Protein standards 2: Non-reduced feedstock B, 3: Reduced feedstock B, 4: Batch folded feedstock B, 5: supernatant after particle collection, 6-10: elution with 500 mM Imidazole, 500 mM NaCl, 20 mM Tris-HCl, pH 8. Molecular weights of standard proteins are shown in the figure and the position of the monomeric HAT-hβ2M molecule is indicated by an arrow.

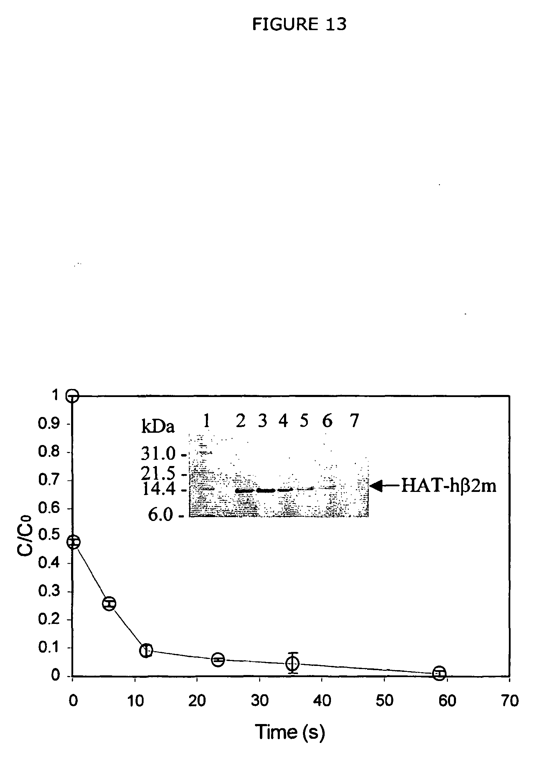

Figure 13. Continuous adsorption of batch folded HAT-hβ2m onto Cu²⁺-charged IDA-linked magnetic supports under laminar flow conditions. The ratio of unbound (C) and bound (C₀) product is plotted against the mixing time. Particle concentration during adsorption was 3.0 mg/ml and the total amount of protein was 100 µg/ml. Batch folding was conducted at a protein concentration of 200 µg/ml and the final urea concentration was 266 mM. The insert shows a SDS-PAGE analysis of collected supernatants after different periods of mixing. Lanes: 1: Protein standards, 2-7: supernatants collected at time points corresponding to the plotted values. Molecular weights of standard proteins are shown in the figure and the position of monomeric HAT-hβ2m is indicated by an arrow.

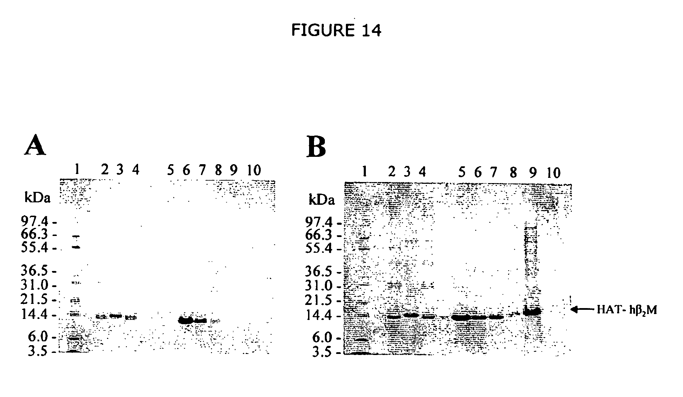

Figure 14. SDS-PAGE analysis of eluted fractions after continuous adsorption of folded HAT-hβ2m from a crude suspension onto Cu²⁺-charged IDA-linked supports. (A) Analysis of collected fractions after elution in aqueous buffer. Lanes: 1: Protein standards, 2: Non-reduced Feedstock B, 3: Reduced Feedstock B, 4: Batch folded suspension, 5: wash supernatant, 6-10: elution with 500 mM imidazole, 500 mM NaCl, 20 mM Tris-HCl, pH 8. (B) Analysis of supernatants collected after the cleaning with denaturing buffers. Lanes: 1: Protein standards, 2: Non-reduced feedstock B, 3: Reduced feedstock B, 4: Batch folded suspension, 5-7: elution with 8 M urea, 500 mM NaCl, 20 mM Tris-HCl, pH 8, 8-10: 25 mM 2-ME, 8 M urea, 500 mM NaCl, 20 mM Tris-HCl, pH 8. Molecular weights of standard proteins are shown in the figure and the position of the monomeric HAT-hβ2M molecule is indicated by an arrow.

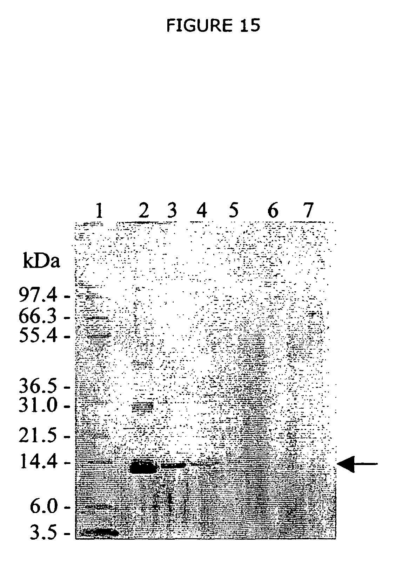

Figure 15. Non-reducing SDS-PAGE analysis of collected fractions during elution of bound HAT-hβ2m from Cu²⁺-charged magnetic chelators in a high gradient magnetic separator. Lanes: 1: Protein standards, 2: Feedstock B, 3-7: Successive elution with 500 mM imidazole in 500 mM NaCl, 20 mM Tris, pH 8. Molecular weights of standard proteins are shown in the figure and the position of the monomeric HAT-hβ2M molecule is indicated by an arrow.

### DETAILED DESCRIPTION OF THE INVENTION

As mentioned above, the present invention i.a. relates to a continuous process for obtaining a preparation of a macromolecular assembly of one or more macromolecular substances including at least one substance selected from the group consisting of proteins, carbohydrates, nucleic acids, nucleic acid analogues, and protein-nucleic acid chimera, said process comprising the steps of:
a) (i) providing one or more fluid compositions each comprising at least one of said one or more macromolecular substances, said one or more macromolecular substances being in a predominantly unassembled form, (ii) providing a dispersion comprising coated, essentially non-porous magnetic particles, and (iii) optionally providing one or more additives;
b) simultaneously or sequentially combining the one or more fluid compositions, the dispersion of magnetic particles, and said one or more optional additives so as to provide a continuous stream comprising said one or more macromolecular substances, the magnetic particles and any optional additives;
c) passing said continuous stream through a first mixing device so as to form magnetic complexes each comprising a magnetic particle and a plurality of macromolecular assembly species;
d) passing the continuous stream through a first capture compartment of a magnetic separator so as to capture said magnetic complexes; and
e) separating said magnetic complexes from said continuous stream, and isolating said macromolecular assembly species from said magnetic particles so as to obtain the preparation of a macromolecular assembly of said one or more macromolecular substances.

It should be understood that the present process is generally continuous in the sense that the "continuous stream" referred to in step (b) is not collected and subsequently batch processed, but is instead passed through the mixing device (step (c)) and subsequently through the magnetic separator (step (d)) in a continuous operation after which the magnetic complexes are separated from said stream (step (e)). It should also be understood that the continuous stream can pass through further devices, pumps, mixers, etc. before and after its passage through the mixing device of step (b). Such devices, pumps, and mixers may be advantageous in order to obtain optimized process parameters in relation to the overall goal of the operation. Thus, the term "continuous" refers to an overall process ongoing over a period of time substantially without interruptions and includes, but is not limited to, terms such as "on-line", "non-stop", "permanent", "constant", "unbroken" and "uninterrupted".

The process allows for continuous assembly of macromolecular substances and complex-formation thereof with magnetic particles followed by capture of the magnetic complexes In a magnetic separator. The macromolecular assembly can subsequently be released from the magnetic complexes.

The aim of the process according to the invention is to obtain a preparation of a macromolecular assembly of one or more macromolecular substances starting from said one or more macromolecular substances in a predominantly unassembled form, i.e. the aim is to obtain a technically valuable macromolecular assembly from a less "organized" starting material. In a number of instances, the "organized" macromolecular assembly exhibits a biological activity, whereas the less "organized" macromolecular substance(s) exhibit(s) no or only limited biological activity. This will be explained further in the following.

The term "macromolecular substances" covers a broad range of commercially and clinically important molecules, such as proteins, carbohydrates, nucleic acids (for example RNA and DNA), nucleic acid analogues (for example PNA (peptide nucleic acids) and LNA (locked nucleic acids)), and protein-nucleic acid chimera.

The molecular weight of the "macromolecular substances" is generally at least 1000 Da, such as at least 2500 Da, or even at least 5000 Da. For proteins, the number of amino acid units is typically at least 10, such as at least 20, or at least 30, or at least 50, or at least 70. Relevant proteins often have a molecular weight of at least 5000 Da, such as at least 10,000 Da. For nucleic acid and nucleic acid analogues, the number of nucleotides (or analogues) is typically at least 6, such as at least 8 or at least 10 or at least 15. Relevant nucleic acids and nucleic acid analogues often have a molecular weight of at least 1000 Da, such as at least 2000 Da. For protein-nucleic acid chimera, the number of nucleotides (or analogues) is typically at least 6, such as at least 8 or at least 10 or at least 15, and the number of amino acid units is typically at least 10, such as at least 20, or at least 30, or at least 50. Relevant protein-nucleic acids chimera often have a molecular weight of at least 5000 Da, such as at least 10,000 Da. Relevant carbohydrates often have a molecular weight of at least 2500 Da, such as at least 5000 Da, or even at least 10,000 Da.

Particularly interesting macromolecular substances are proteins. The present invention has primarily been illustrated with reference to proteins (see the Examples), but extension to the other types of macromolecular substances will be straightforward for the person skilled in the art.

As used herein, the term "macromolecular assembly" refers to an "organized" structure comprising a single macromolecular substance or a plurality of macromolecular substances. A "macromolecular assembly" is typically more functional, or can exhibit a more refined functionality, compared to the one or more macromolecular substances in a predominantly unassembled form. A "macromolecular assembly" is typically capable of performing a biological function (e.g. a biological effect or a function relevant for a biological system), e.g. as assessed by an *in vitro* assay or any other standard methods known by those skilled in the art. Typically, it is desirable to assemble a singe type of macromolecular assembly, although the process of the invention in principle allows for the assembly of multiple types of macromolecular assemblies.

The term "macromolecular assembly species" refers to a single macromolecular assembly.

The term "predominantly" refers to a degree of at least 60%, i.e. "predominantly unassembled form" refers to a preparation wherein at least 60% of the macromolecular substances (by number) are present in the undesirable unassembled form. When referring to macromolecular substances that exhibit a biological activity when present in the "assembled form", i.e. the macromolecular assembly, the term "predominantly unassembled form" often reflects that said one or more macromolecular substances exhibit less than 40% of the maximum biological activity, and typically the substances exhibit less than 10%, or even less than 5% of the maximum biological activity, or (most typically) virtually no biological activity.

The above-mentioned macromolecular substances are initially present in a so-called "unassembled form", e.g. in the form of unfolded (denatured), misfolded or aggregated, non-hybridized or non-functional form. For proteins, the "unassembled form" is typically a biologically inactive form, e.g. an unfolded (denatured), misfolded or aggregated form, e.g. inclusion bodies, aggregates, precipitates, intermolecular complexes, intramolecular complexes, vaccine vectors/carriers and random coils. For nucleic acids, nucleic acid analogues and protein-nucleic acid chimera, the "unassembled form" is typically the non-hybridised form.

The process for assembling the one or more macromolecular substances can be at least partly self-driven or spontaneous, or can be assisted or mediated by added additives such as buffers, enzymes, biological catalysts, inorganic catalysts, chaperones and reactive chemical reagents.

Also the process parameters in any mixers, reactors and conduits throughout the process system (i.e. not only in steps (b) and (c)) may be controlled and adjusted so as to facilitate assembly, e.g. the assembly conditions may be controlled with respect to physical or thermodynamic parameters such as volume, flow of reactants and buffers, temperature and pressure, and/or with respect to chemical parameters such as pH, ionic strength, redox potential, and content of surfactants, protease inhibitors, ATPase inhibitors, etc. Examples of the latter inhibitors are protease inhibitor(s) selected from the group consisting of peptides, proteins, N-ethyl-maleimide, pepstatin, phenyl methyl sulphonic flouride (PMSF) and EDTA, respectively, and of ATP dependent proteolysis inhibitors such as sodium ortho vanadate. The assembly conditions may further be controlled with respect to enzymatic parameters such as presence of heat-shock proteins, oxidizing or reducing enzymes, peptidyl prolyl cis-trans isomerases and disulfide isomerases. The chemical and enzymatic parameters may be adjusted by addition of the one or more additives.

For proteins, the formation of a desirable tertiary structure for a single macromolecular substance or a quaternary structure for two or more macromolecular substances typically involves formation of a certain number of hydrogen bonds, a number of hydrophobic interactions and possibly a number of sulfur bridges (-S-S-). The formation of hydrogen bonds is often pH dependent, whereas the formation of sulfur bridges often requires a certain redox potential. The establishment of a certain pH or a certain redox potential can typically be obtained by additions of suitable additives, such as buffers, oxidants and reducing agents.

The number of macromolecular substances needed for the establishment of the macromolecular assembly is typically 1-100, such as 1-50, and more typically 1-20. For proteins, the number is typically 1 or 2-10.

In one embodiment, only one macromolecular substance is required for the formation of the macromolecular assembly. In a specific embodiment hereof, the macromolecular substance is represented by a single protein, the unassembled form of said protein being the unfolded, misfolded or aggregated form, and the molecular assembly of said protein being the biologically active, folded form. In a further embodiment, the macromolecular assembly consists of a recombinantly prepared protein.

In another embodiment, at least two macromolecular substances are required for the formation of the macromolecular assembly. Thus, a corresponding number of macromolecular substances should be provided in step (a), either as one single fluid composition or, possibly more typical, as a number of fluid compositions corresponding to the number of macromolecular substances.

In one specific embodiment thereof, said at least two macromolecular substances are represented by a plurality of proteins, the unassembled form of said proteins being the respective proteins in unfolded, misfolded or aggregated form, and the macromolecular assembly of said proteins being a biologically active, quaternary structure wherein the respective proteins represent subunits or domains.

In another specific embodiment thereof, said at least two macromolecular substances are represented by a plurality of species selected from nucleic acids, analogues of nucleic acids, and protein-nucleic acid chimera, the unassembled form of said substances being the non-hybridized forms of said species, and the molecular assembly of said substances being the mutually hybridized form of said species.

### The Process - Step (a)

The process comprises a first step of (i) providing one or more fluid compositions each comprising at least one of said one or more macromolecular substances (1), said one or more macromolecular substances being in a predominantly unassembled form, (ii) providing a dispersion comprising coated, essentially non-porous magnetic particles (8), and (iii) optionally providing one or more additives (2).

### Fluid composition comprising macromolecular substance(s)

The macromolecular substances include at least one substance selected from the group consisting of proteins, carbohydrates, nucleic acids, nucleic acid analogues, and protein-nucleic acid chimera. Typically, all macromolecular substances are selected from the group members above. Furthermore, in many embodiments, the macromolecular substances are of the same type, i.e. all are proteins, or all are nucleic acids, nucleic acid analogues or protein-nucleic acid chimera, or all are carbohydrates, etc.

The macromolecular substances are typically dissolved or suspended in a liquid so as to form a fluid composition thereof. The concentration of unassembled macromolecular substance(s) in a given liquid may be less than 100 mg/ml, 10 mg/ml or less than 1 mg/ml, including less than 300 µg/ml, such as less than 100 µg/ml, including less than 30 µg/ml, 10 µg/ml, 3 µg/ml, 1 µg/ml, such as less than 300 ng/ml, including less than 100 ng/ml, 30 ng/ml, or even less than 3 ng/ml.

The liquid is typically water or an aqueous mixture/solution, such as pure water, an aqueous buffer, a water/ethanol mixture, a water/DMSO mixture, or an aqueous salt solution, e.g. saline, an urea solution or guanidine solution. A suitable aqueous liquid may also comprise a surfactant.

In one embodiment, the macromolecular substance(s) is/are selected from the group consisting of nucleic acids, nucleic acid analogues and protein-nucleic acid chimera.

In another embodiment, the macromolecular substance(s) is/are proteins.

In a particular embodiment, the one or more unassembled macromolecular substance is in a denatured form. As examples hereof, the substances have been denatured by non-chemical means such as with a temperature or pressure change, or the substances have been denatured by adding a compound selected from the group of organic solvents, chaotrophic agents, detergents, and salts. Examples of chaotrophic agents are urea and guanidine hydrochloride, wherein the concentration of urea or guanidine hydrochloride is typically in the range of 0.1-9 M such as 5-7 M including approximately 6 M.

The origin of the unassembled macromolecular substances can be fairly diverse, however such substances (in particular proteins, carbohydrates, nucleic acids, nucleic acid analogues, and protein-nucleic acid chimera) are typically derived from a vertebrate including the group consisting of humans, animals and murine species, a rat species, a porcine species, a bovine species, a fish species and an avian species, or is derived originally from a non-vertebrate species including the group of eukaryotes, prokaryotes and archaebacteria, including bacteria, yeast, fungus, virus, prions, plants, insects and spiders.

In one embodiment, the fluid composition is taken directly from a fermentation broth. Within this embodiment, the macromolecular substance(s) is/are typically a recombinant protein.

### Dispersion of magnetic particles

A dispersion comprising coated, essentially non-porous magnetic particles must also be provided. The term "particle" should be read in the broadest term and includes beads, amorphous particles, crystals, filaments, etc. Structurally, the magnetic particles differ from conventional chromatographic supports by generally having a size in the micron to submicron range and a base matrix, which is essentially non-porous to the macromolecular substances. It is to be understood that any particle possessing magnetic properties/susceptibilities can be used in conjunction with the present invention. Another important feature of the coated, essentially non-porous magnetic particles is that the tendency to become clogged with biological foulants is minimized. Support fouling is a major problem with conventional porous chromatographic supports. Furthermore, the particles possess magnetic properties, preferably superparamagnetic properties, i.e. they have no magnetic memory. Superparamagnetic properties provide a number of advantages: (i) prevents aggregation of the particles and (ii) ensures easy and quick release of the particles from the magnetic separator once the field has been removed or switched off.

In one embodiment, the magnetic particles form a magnetic ferro-fluid.

In a preferred embodiment, the magnetic particles are superparamagnetic.

Particles can be coated with an organic or inorganic molecule that can form polymers on the surface of the particles, such as but not limited to poly-acrylamide, poly-glutaraldehyde and agarose or dextran, and this coating can be used as a scaffold for particle activation and functionalisation. Depending on the macromolecular assembly to be captured by the particles, the person skilled in the art will be able to select appropriate molecules for the coating procedure and suitable methods to perform this operation are described in the art.

The coating of the essentially non-porous magnetic particles needs not necessarily to be non-porous, but may be at least partly porous to the macromolecular substances. In some embodiments, however, the coating as such is also essentially non-porous to the macromolecular substances. In other embodiments, the coating as such is porous to the macromolecular substances.

Preferably, the coating of the particles is thin, which facilitates fast adsorption kinetics due to low mass transfer resistance. The term "thin" refers to the fact that the coating only represent up to 20% of the diameter of the particles, such as up to 10% of the diameter of the particles.

Generally, methods for preparation of magnetic particles are described in the art and the person skilled in the art will be able to select and test appropriate combinations of coating, activation, and coupling chemistries.

In a specific embodiment of the present invention magnetotactic bacteria, such as but not limited to *Magnetospirillum,* expressing suitable surface exposed ligands, could be used as the capturing agent in the present invention. The surface functionality of the bacteria could be modified in order to either capture the assembled or the unassembled molecule.

The size (average diameter) of the magnetic particles is typically at least 1 nm, such as at least 0.01 µm, at least 0.1 µm, at least 1 µm, at least 5 µm, at least 10 µm, at least 20 µm, at least 30 µm, at least 40 µm, at least 50 µm, at least 100 µm, at least 150 µm or at least 200 µm, e.g. in the range of 1 nm to 250 µm. The size distribution of particles needs not necessarily to be narrow. It is envisaged that a fairly broad particle size distribution (e.g. covering the range: average diameter ± 50%) also may provide suitable results.

In a further embodiment, magnetic particle batches representing two or more different nonoverlapping size ranges are used. In this embodiment, it is envisaged that the particle batches may have different coatings and/or different ligands so as to bind different components. As an example, one batch may form complexes with the macromolecular assembly, whereas another batch may bind certain bi-products (e.g. contaminants, enzymes, undesirable proteins, etc.) An illustrative example is a process where a preparation of a protein-containing macromolecular assembly is obtained from a fermentation broth. A fermentation broth typically comprises other enzymes and proteins than that/those of the macromolecular assembly, and it would therefore be desirable to bind such enzymes/proteins to one particle batch (e.g. a batch of "larger" magnetic particles) and bind the target molecular assembly to another particle batch (e.g. a batch of "smaller" magnetic particles).

In the foregoing embodiment, it is also envisaged that capture of the magnetic particles/magnetic complexes may be effected by two or more magnetic separators arranged in series so that a first batch of ("larger") magnetic particles/magnetic complexes is captured in the capture compartment of a first magnetic separator, and a second batch of ("smaller") magnetic particles/magnetic complexes is captured in the capture compartment of a second magnetic separator. The magnetic field should of course be adjusted so that the small particles of the second batch are not captured in the compartment of the first magnetic separator.

In order to facilitate the formation of complexes with the macromolecular substances or macromolecular assembly, the magnetic particles should be able to bind the macromolecular substances/macromoleclar assembly sufficiently strongly. The binding should on the other hand be reversible so that the molecular assembly subsequently can be isolated from the magnetic particles. The magnetic particles typically carry one or more types of ligands. The ligand chemistry of the magnetic particles can be selected by the person skilled in the art depending on the type of macromolecular assembly to be separated. Ligand chemistries include affinity, immobilized metal chelate affinity, antibody affinity, streptavidin-biotin affinity, pseudo affinity, chromatofocusing, ion exchange, tentacle chromatography, hydrophobic interaction, mixed mode and hydrophobic charge induction.

In one embodiment, the selected ligand(s) has/have affinity for at least one of said one or more macromolecular substances.

In another embodiment, the selected ligand(s) has/have affinity for the macromolecular assembly. In a variant hereof, the selected ligand has substantially no affinity for the unassembled, individual macromolecular substances.

It should be understood that the present invention is not limited to magnetic particles carrying specifically introduced ligands, on the contrary, the present invention is applicable as long as the magnetic particles has affinity for or adsorbs the macromolecular assembly, or at least one of said one or more macromolecular substances.

### Additives

The one or more optional additives are selected with the aim of optimizing the chemical and enzymatic process parameters (see above). Examples hereof are buffer components, salts, organic and inorganic compounds, and surfactants.

### The Process - Step (b)

A further step of the process comprises simultaneously or sequentially combining the one or more fluid compositions (1), the dispersion of magnetic particles (8), and said one or more optional additives (2) so as to provide a continuous stream comprising said one or more macromolecular substances, the magnetic particles and any optional additives.

Combination of the various constituents of the stream can in principle be effected either simultaneous or in any sequential order.

In one embodiment of the invention, the one or more fluid compositions and the one or more optional additives are combined and passed through a second mixing device so as to form a continuous pre-stream of the macromolecular assembly of the one or more macromolecular substances, said pre-stream subsequently being combined with the dispersion of magnetic particles so as to form the continuous stream. In this instance, it is advantageous that the ligands of the magnetic particles are chosen so that binding to the macromolecular assembly is favoured over binding to the individual macromolecular substances. Furthermore, it is preferred that the pre-stream is passed through a second mixing device so as to ensure proper contact between the fluid compositions and the one or more optional additives before contact with the magnetic particles. The second mixing device can be selected following the directions for the first mixing device (see "The Process - Step (c)" below). It should be understood that, in a variant of this embodiment, at least one of the one or more optional additives is added together with the magnetic particles

In another embodiment, at least one of the one or more fluid compositions and the dispersion of magnetic particles are combined and passed through a second mixing device so as to form a continuous pre-stream of magnetic pre-complexes of magnetic substances and one or more macromolecular substances, said pre-stream subsequently being combined with any remaining of the one or more fluid compositions and the one or more optional additives so as to form the continuous stream. In this instance, it is advantageous that the ligands of the magnetic particles are chosen so that binding to one of the macromolecular substances is favoured, so that complexation between the one macromolecular substance and a magnetic particle occurs before assembly of the macromolecular assembly. Furthermore, it is preferred that the pre-stream is passed through a second mixing device so as to ensure proper contact between the fluid composition(s) and the magnetic particles before contact with remaining of the one or more fluid compositions and the one or more optional additives. The second mixing device can be selected following the directions for the first mixing device (see "The Process - Step (c)" below). It should be understood that, in a variant of this embodiment, at least one of the one or more optional additives is added upon preparation of the pre-stream.

In the before-mentioned embodiment, the assembly process is initiated directly on the magnetic particles, meaning that instead of forming the macromolecular assembly in solution followed by complexation with the magnetic particles and then separation in a magnetic separator, the at least one unassembled macromolecular substance is bound to the magnetic particles followed by assembling of the macromolecular assembly directly on the magnetic particles and purification of the magnetic complexes in a magnetic separator. It is to be understood that any part of the assembly process could be conducted in solution followed by adsorption/binding to the magnetic particles followed by addition of further unassembled macromolecular substances and/or additives conditions to complete the assembly process. The following non-limiting example is included to further explain this particular embodiment: A fluid composition comprising an unassembled macromolecular substance is mixed with a dispersion of magnetic particles in a second mixing device, leading to formation of a magnetic pre-complex of the macromolecular substance and a magnetic particle. Another stream containing a second unassembled macromolecular substance is introduced into the fluid stream containing said magnetic pre-complex and mixed in a first mixing device, resulting in the formation of magnetic complexes of a macromolecular assembly and magnetic particles. The stream containing the magnetic complexes is passed directly through the capture compartment of a magnetic separator and the magnetic complexes are captured in the separator. Subsequently, washing and elution buffers are introduced into the capture compartment and the magnetic field is removed and re-applied as convenient, leading to isolation of the macromolecular assembly from the magnetic particles. Particles and assembled product can be collected separately.

In a still further embodiment, the one or more fluid compositions, the dispersion of magnetic particles, and one or more optional additives are combined substantially simultaneously so as to form the continuous stream.

### The Process - Step (c)

A further step of the process comprises passing said continuous stream through a first mixing device (10,11) so as to form magnetic complexes each comprising a magnetic particle and a plurality of macromolecular assembly species. The aim of this process step is to ensure that assembly of the macromolecular substances and the formation of the magnetic complexes proceeds efficiently. This is preferably realized by a mixing device facilitating turbulent mixing conditions for the constituents of the continuous stream. It is, however, envisaged that assembly of certain substances which are susceptible to high shear forces may require a laminar (non-turbulent) flow.

The term "magnetic complex" is intended to mean a structure comprising a magnetic particle and a plurality of macromolecular assembly species. Thus, it is envisaged that one magnetic particle has bound thereto a plurality of macromolecular assembly species. The binding is typically obtained by intermolecular forces such as hydrogen bonds and hydrophobic interactions. The binding preferably involves an interaction between ligand(s) on the surface of the magnetic particles and corresponding groups or receptors in the macromolecular substances and/or the macromolecular assembly. Thus, the binding is preferably substantially reversible.

Mixers and/or pipe reactors can be selected from the group consisting of inline static mixers, static mixers, motionless mixers, top entry mixers, side entry mixers and sanitary mixers, but is not limited hereto as any conceivable mixing device capable of producing the conditions leading to formation of the assembled product should be considered part of the present invention. Mixers operated by electrical and/or mechanical, magnetic or ultrasonic means can be used in conjunction with the present invention.

In one embodiment, the mixing device comprises an inline static mixer (11). In alternative embodiments, the mixing device is selected from the group consisting of mechanical, magnetic or ultrasonic mixers.

### The Process - Step (d)

A further step of the process is the passing of the continuous stream through a first capture compartment (16) of a magnetic separator (15) so as to capture said magnetic complexes. The aim is to capture the magnetic particles and thereby the macromolecular assembly.

The magnetic separator (15) typically comprises one or more capture compartments (16) through which the continuous stream is passed through whereby the magnetic complexes are captured. Illustrative examples of such magnetic separators are high gradient magnetic separators, open gradient magnetic separators, superconductor magnetic separators, and continuous carousel separators, all of which are particularly useful for capture of small size magnetic particles.

The magnetic separator is preferably equipped with a mechanism allowing the magnetic field in the capture compartment to be partially or substantially switched off. Thus, the magnetic separator preferably has at least two operational modes, a first of the at least two operational modes providing a magnetic field to the first capture compartment (16) thereby rendering the first capture compartment (16) capable of capturing the magnetic complexes (magnetic particles) (8), and a second of the at least two operational modes providing an inadequate magnetic field to the first capture compartment (16) thereby rendering the capture compartment (16) substantially incapable of first capturing magnetic complexes (magnetic particles) (8).

In a specific embodiment, magnetic separator is equipped with a reciprocating separator canister.

In a specific embodiment of the present invention, the magnetic separator is a continuous carousel separator, which allows the entire process, including batch cycles of washings, elution and cleaning, to be performed in continuous mode. This is achieved by having a number of canisters/filter chambers (first capture compartments (16)), such as for example 36, arranged radially on a rotating carousel and covered by several permanent magnets, such as for example three. Several of these chambers are then filled with the magnetic complexes and once filled, the chambers are rotated away from the permanent magnet allowing the captured magnetic complexes to be released from the chambers, e.g. into a recycle loop as described elsewhere herein. In this way it is possible to load, wash and recover the macromolecular assembly and magnetic particles while at the same time other canisters (second capture compartments) are being filled with the magnetic complexes.

It is to be understood that any separation technique utilizing magnetic forces to a significant degree to collect the magnetic complexes can be used in conjunction with the present invention. Furthermore, separation methods combining magnetic forces with other forces, including gravitational forces, are applicable to the present invention.

### The Process - Step (e)

The process according to the invention further comprises the step of separating said magnetic complexes from said continuous stream, and isolating said macromolecular assembly species from said magnetic particles so as to obtain the preparation of a macromolecular assembly of the one or more macromolecular substances.

Separation of the magnetic complexes from the continuous stream allows for handling of the magnetic complexes, e.g. removal of the magnetic complexes from the magnetic separator, and/or liberation of the predominantly assembled macromolecular substances from the magnetic particles, e.g. by elution.

One way of effecting that the separation of the magnetic complexes from the continuous stream is by redirecting said continuous stream to a second capture compartment. If a magnetic separator with a plurality of capture compartments is used, the continuous stream may be alternated between capture compartments. Thus, in one embodiment, the second compartment is a second compartment of the magnetic separator also having the first capture compartment. The continuous carousel separator mentioned above is one embodiment of such a magnetic separator wherein certain sets of compartments are in operation with respect to the continuous stream, and other sets of compartments are in operation with respect to washing fluids, eluents, etc.

In a more simplified set-up, the magnetic complexes are simply separated from the continuous stream by interrupting (or even terminating) said stream. In this embodiment, the capture compartment of the magnetic separator may simply be flushed with a washing fluid subsequent to interruption/termination of said stream so that the magnetic complexes have no longer contact with the stream or the fluid representing the stream.

The product of the process, namely the preparation of the macromolecular assembly, can be isolated by eluating the magnetic complexes with a suitable eluent. This can be effected while the magnetic complexes are still present in the capture compartment, or the magnetic complexes may initially be removed from the capture compartment before eluation. This is further explained herein and in the examples.

The yield of the process is at least 1 ng, at least 1 µg, at least 0.1 mg, at least 1 mg, at least 10 mg, 100 mg, and as the process is applicable for large scale commercial processes, the yield may even be at least 1 g, at least 10 g, at least 100 g, at least 1 kg, at least 10 kg, at least 100 kg or even at least 1t.

### Optional further steps

It is envisaged that the continuous stream, after having been deprived of the magnetic complexes in step (d), still contains valuable constituents such as buffer components, small amounts of unassembled macromolecular substances, and small amounts of non-complexed macromolecular assembly.

Thus, in one embodiment, at least a fraction (e.g. 5-80%, such as 10-50%) of the continuous stream, after passage through the capture compartment of the magnetic separator, is continuously fed back upstream so as to become a part of the continuous stream of step (b). Depending on the actual content of the continuous stream, the "back feed" may be combined with any of the one or more fluid compositions and the optional additives, or the "back feed" may be fed upstream relative to any of the mixers (10,11,5,6) involved in the process.

Also, it is envisaged that the magnetic particles can be recycled, e.g. fed back upstream and mixed with the dispersion of magnetic particles or fed upstream to any of the mixers (10,11,5,6) involved in the process, after isolation of the macromolecular assembly.

Further, it should also be appreciated that this invention is not only suitable for large-scale production of a macromolecular assembly, but also for microfluidic scales as the magnetic separator principle disclosed here offers scale-flexibility in both directions, mainly due to the small size of the magnetic particles used in conjunction with the invention. Furthermore, one could also envisage the synthesis of proteins form amino acids and formation of specific sequences of RNA/DNA from nucleotides being adapted to the invention. This could be done for example by immobilizing the part of the macromolecular substance onto the magnetic particles (in very small scale, it could be possible to use only one particle) followed by continuous addition of subunit structures such as amino acids and/or nucleotides followed by magnetic capture of the product-loaded particles. Methods for immobilization are described in the art. Another possibility is to allow the synthesis of the molecules, mentioned above, in solution followed by magnetic capture as described previously for other types of macromolecular assemblies.

As an alternative to the main aspect of the present invention can be mentioned the variant where the ligand chemistry of the magnetic particles is selected in such a way that the unassembled macromolecular substances and/or other contaminants are adsorbed to the particles and the macromolecular assembly is not. In this way, the by-products are removed in the magnetic separator, and the product of interest (i.e. the macromolecular assembly) is collected in the exiting liquid from the magnetic separator. This variant otherwise follows the directions given herein, *mutatis mutandis.*

### The system

The present invention also provides a system, which is particularly useful for exploiting the process defined above. Thus, the present invention provides a system useful for the continuous assembly and capture of macromolecular substances, the system comprising: a plurality of containers including at least one first container containing a dispersion of coated, essentially non-porous magnetic particles (8), at least one second container containing a liquid composition comprising one or more macromolecular substances (1), and optionally at least one third container (2) containing one or more additives; at least one pumps (3,4,7) for causing the content of each of said containers (1,2,8) to be fed to a first mixing device (10,11); a conduit for providing passage from said first mixing device (10,11) to the capture compartment (16) of a magnetic separator (15), said magnetic separator (15) having at least two operational modes, a first of the at least two operational modes providing a magnetic field to the first capture compartment (16) thereby rendering the first capture compartment (16) capable of capturing the magnetic particles (8), and a second of the at least two operational modes providing an inadequate magnetic field to the first capture compartment (16) thereby rendering the first capture compartment (16) substantially incapable of capturing the magnetic particles (8).

In a preferred embodiment of the system, the first mixing device (10,11) comprises at least one first pipe reactor (11) for facilitating turbulent mixing conditions for the content of said containers (1,2,8).

In a further embodiment, the system further comprises a second mixing device (5,6) arranged downstream relative to the at least one second container containing the one or more macromolecular substances (1) and the at least one third container containing one or more additives (2), and upstream relative to said first mixing device. More particularly, said second mixing device (5,6) comprises at least one second pipe reactor (6) facilitating turbulent mixing conditions for the combined content of said second and third containers fed thereto.

In a still further embodiment, the system further comprises a second mixing device (5,6) arranged downstream relative to the at least one first container containing the dispersed magnetic particles (8) and the at least one second container containing the one or more macromolecular substances (1), and upstream relative to said first mixing device. More particularly, said second mixing device (5,6) comprises at least one second pipe reactor (6) facilitating turbulent mixing conditions for the combined content of said second and third containers fed thereto.

In a further embodiment, the system further comprises at least one recycle conduit (23,24, 25) for providing a passage from the capture compartment (16) to a system member upstream relative to the first mixing device (10,11). In this embodiment, it is envisaged that the magnetic particles can be recycled, e.g. fed back upstream via a recycle conduit (23,25) and mixed with the dispersion of magnetic particles or fed upstream via a conduit (23) to any of the mixers (10,11,5,6) involved in the process, after isolation of the macromolecular assembly.

Embodiments of the system are illustrated in Figures 1, 2 and 11.

An embodiment of the process and system of the invention can be described as follows: Macromolecular assembly (see Figure 1 or Figure 2) is initiated by combining at least two fluid compositions (1,2), in which at least one contains an unassembled macromolecular substance (1), in a mixing device (5,6), such as an inline static mixer. The mixing device (5,6) facilitates formation of a macromolecular assembly from the macromolecular substances. Conditions for macromolecular assembly can be controlled by adjusting the flow rates of the pumps (3,4) and maintained throughout the process, as the reaction is run continuously.

Additional control of the reaction conditions can be added into the system by adjusting the concentration of unassembled macromolecular substance(s) in the fluid composition(s) (1). Furthermore, different additives (2)(e.g. buffers, surfactants, etc.) can be added together with the fluid composition(s) into the mixing device (5,6) in order to ensure proper conditions for macromolecular assembly. Sufficient retention time for the formation of the macromolecular assembly can be obtained by increasing the length and/or size of the inline static mixer (6). Depending on the macromolecular assembly, the number of fluid compositions (1,2) and pumps (3,4) can be varied to generate the required conditions. These conditions can be tested and selected by the person skilled in the art using known standard methods.

After the formation of at least part of the macromolecular assembly, a dispersion of magnetic particles (8) is introduced, preferably via a pump (7), into the liquid stream containing the macromolecular assembly, and the two streams are mixed in another mixing device (10,11), which ensures proper fluid conditions for formation of magnetic complexes. Since the appropriate conditions for the formation of the magnetic complexes might differ from the optimal conditions for the assembly reaction, buffer components (e.g. salts, organic and inorganic compounds) and/or other additives, including surfactants, can be introduced (not shown in Figure 1) into the liquid stream containing the assembled product at any point between the second mixing devices (5,6) and first mixing devices (10,11). Preferably, these changes involve modifications of either conductivity and/or pH. Particle concentration during mixing can be adjusted by changing the concentration of particles in the dispersion (8) and/or by changing the fluid velocity of the pump (7). The ratio of macromolecular assembly to magnetic particles can be specified from a knowledge of the efficiency of the assembly reaction in question combined with the flexibility of the following parameters of the process: i) the speed of the pumps (3,4,7) and ii) particle concentration in the dispersion (8) and concentration of macromolecular substances in the suspensions (1) entering the system. Furthermore, the necessary time to form a sufficient amount of magnetic complexes prior to separation in the magnetic separator (15) can be set by changing the volume and/or length of the mixing device (10,11).

Subsequently, the liquid flow, containing the magnetic complexes, is directed through a magnetic separator (15). The magnetic field in the capture compartment (16) of the magnetic separator (15) should usually be applied perpendicular or parallel to the fluid flow direction and should be strong enough to retain the major part of the magnetic complexes in the separator, whereas the liquid runs freely through the separator. This set-up allows soluble and insoluble by-products to be separated from the assembled product, as they are free to run through the separator. The exiting liquid can either be collected (22) or re-cycled (23,24) back into the system, to reduce buffer consumption.

Once the particles are captured inside the capture compartment (16) and the stream is redirected or interrupted, a number of solutions/buffers (19) can be introduced into the magnetic separator to remove unbound macromolecular substances and/or other contaminants, elute the macromolecular assembly and clean the magnetic particles. If necessary other additives could be added into the system. In a specific embodiment, different enzymes are added into the separator to modify the macromolecular assembly. Examples of modifications include proteolytic change, such as removal of a tag sequence or a fusion product, and protein disulphide formation. Pumps to control the liquid flow of the solutions/buffers into the magnetic separator (not shown in Figure 1) could be placed between the buffer reservoirs (19) and the magnetic separator (15).

Operations of particle washing, product elution and particle cleaning inside the separator (15) can be performed in number of different ways such as for example: i) magnetic complexes are released into a recycle loop (see Figure 2) inside the separator (15) by removing or switching off the magnetic field and are mixed with appropriate buffers (19) in said recycle loop (particles are then re-captured by re-applying the magnetic field), ii) magnetic complexes are mixed with the buffers directly between the magnetic poles of the separator, and thorough mixing can then be achieved by either continuously switching off and on the magnetic field or by shaking the tubing or canister (not shown in Figures 1 and 2) by mechanical or ultrasonic means. Releasing the particles into a recycle loop probably produces the best possible mixing conditions, but suffers from the inherent drawback of diluting the particle suspension. This can be avoided by mixing the particles with buffers directly inside the chamber positioned between the magnetic poles, leading to higher product concentration factors.

Washings, unassembled macromolecular substances and cleaning solutions can be collected (22) or recycled through the system. In a specific embodiment of the present invention unassembled macromolecular substances are washed away from the magnetic particles during cleaning in the magnetic separator and are recycled through the system (23,24) in order to increase the overall recovery of the assembled product.

Retained particles can be released inside the magnetic separator by removing or switching off the external field and can be flushed out of the separator by pumping buffer/solution (19) through it. Released particles can either be collected (22) or recycled through the system (23,25).

Different valve types and settings can be used to direct the particle suspension and buffers/solutions through the canister and the recycle loop (not shown in Figure 1). Preferably electrical, pneumatic or mechanical 3-way valves are used, but other valves, including multiple way valves, solenoid valves, manual valves, pressure valves and shutoff valves, can also be applied.

In summary, the present invention provides a method and a system for continuously assembling macromolecular substances. Batch reaction steps are avoided as the assembly process and the following adsorption/binding to magnetic particles take place under continuous conditions in a flow-through mixing device. In this way, the macromolecular assembly can be efficiently isolated in one single cycle and the problems associated with mixing of large volumes is circumvented. The use of a magnetic separator allows particulate by-products formed as a result of inefficient assembly to pass through the separator, thereby bypassing the need for filtration and centrifugation prior to primary capture of the product. By combining macromolecular assembly, capture, concentration, clarification and initial purification, the number of unit operations can be significantly reduced, resulting in higher throughput, higher yields and better overall process economy compared to conventional processes.

### EXAMPLES

In the following, a number of examples are provided to illustrate how the present invention can be exploited. The first example is designed to comprehensively demonstrate how the invention can be used for assembly of a particular macromolecular substance (a protein). Subsequent examples demonstrate specific aspects of the invention. For all these examples we have chosen to use the protein refolding reaction (a simple type of assembly reaction) of the monomeric fusion molecule HAT (histidine affinity tag) human β2-microglobulin, which was produced as insoluble inclusion bodies in *E. coli.* We have used metal chelate affinity ligands coupled to coated, essentially non-porous superparamagnetic particles to capture the assembled product followed by separation and purification in a magnetic separator. The metal chelate affinity supports were charged with copper to provide a tight interaction between the product and the particles. It is to be understood that these examples are not intended to limit the scope of the present invention in any manner.

### Example 1: Assembly of active HAT human β2-microglobulin from inclusion bodies.

Below, an example is given of how the invention can be used. Firstly, we describe one possible process set-up exploiting high gradient magnetic separation to capture product-loaded particles. Then we demonstrate how the invention is used in practice for the assembly of correctly folded and active human β2-m.

### Materials and methods

### Isolation and solubilisation of inclusion bodies

HAT-hβ2m was produced as insoluble inclusion bodies by *E. coli* fermentations in a 2 L Labfors fermentor (Ingfors AG, Bottmingen, Switzerland) as described by Ferre *et al.,* (2003). Intracellular inclusion bodies were released, washed and solubilised as described by Ferré *et al.,* (2003). Two batches of denatured inclusion body-derived HAT-hβ2m were prepared and designated Feedstock A and B, respectively. Both stocks were solubilised in 8 M urea in 20 mM Tris-HCl, pH 8. Feedstock A has a total protein concentration of 3 mg/ml and contains approximately 50-60% monomeric HAT-hβ2m, whereas Feedstock B is 10 times more concentrated (30 mg/ml), containing 80% monomeric HAT-hβ2m.

### Preparation of coated, essentially non-porous superparamagnetic adsorbents

The base matrix of the superparamagnetic particles was prepared as described by Hubbuch *et al.,* (2002). These particles were coated with an ultra-thin layer of poly-glutaraldehyde as described by O'Brien *et al.,* (1996) and Zulqarnain (1999). Poly-glutaraldehyde particles were activated with allyl glycidyl ether as described by Burton and Harding (1997) and Heebøll-Nielsen (2002). In this procedure, the poly-glutaraldehyde particles are activated with the 6 C-atom hydrophilic spacer arm allyl glycidyl ether (AGE), which is then coupled with iminodiacetic acid (IDA). IDA groups were coupled to the AGE-activated supports by substituting the bromine ion on the spacer arm as described by Heebøll-Nielsen *et al.,* (2003). Prior to use, the IDA-linked supports were charged with 0.1 M CuSO₄ and equilibrated with binding buffer (0.5 mM NaCl in 20 mM Tris-HCl, pH 8). Particle concentration was determined by the dry-weight method described by Hubbuch, (2000).

### System setup for magnetic adsorbent mediated process for continuous refolding and on-line separation

A schematic diagram of the system setup is depicted in Figure 2 and the technical elements of the process are described in detail below.

Pumps: The flow rate of the denatured HAT-hβ2m suspension (1) was controlled by a P1 peristaltic pump (3) (Amersham Biosciences, Sweden) and all other pumps (4, 7, 13) were model 503U peristaltic pumps (Watson-Marlow, England).

Mixers: The protein folding reaction was initiated in a Y-mixer (5) and the particle suspension (8) was introduced into the protein stream via a F-shaped mixing block (10). Both mixers had inlet and outlet channels with an inner diameter (i.d.) of 1 mm. Magnetic particles were kept In suspension using a motor driven two-bladed impeller (9) (Heidolph Elektro KG, type RZR, Germany).

Valves: All manual 3 way valves (12, 17, 18, 20) were made of teflon and had channels with internal diameters of 2 mm (Kebo Lab A/S, Denmark).

Folding and adsorption pipe reactors: The folding pipe reactor (6) was made up of 1 mm silicone tubing and the adsorption pipe reactor (11) was constructed by twisting hard teflon tubing (i.d. = 2 mm) at every 50 mm to generate turbulent mixing conditions.

Magnet: Product-loaded particles were retained in the filter canister (16) using a Steinert HGF 10^{®} separator (15), equipped with a switchable magnet (Hoffman *et al.,* (2001). Rotation of the HGF 10 magnet in the iron yoke, makes it possible to switch the magnetic field on (corresponding to position 90° and 270°) and off (corresponding to positions 0/360°). Field levels at the 4 positions 0/360°, 90°, 180° and 270° were 18.4 mT, 0.56 T, 29.5mT and 0.55 T, respectively. Particle capture was conducted with the magnet positioned at 90°.

Magnetic filter: A cylindrical canister (16) was packed to 10% of its total volume with a mesh (Fibres of stainless steel 430, with a diameter of 110 µm) resulting in a total void volume of 3.89 mL. The canister was placed vertically in the air gap (width 1.5 cm) between the poles of the magnet.

Fraction collector: Fractions (22) were collected using a fraction collector model CDM210 (LKB Bromma, Sweden).

### Operation of the HGMS system during continuous protein folding and product purification

Operation steps during the process are explained with reference to Figure 2.

Continuous folding and magnetic adsorption: Protein folding was initiated by continuous dilution of the denatured protein solution (1) with binding buffer (2) in the first mixing chamber (5). Passage through the folding reactor (6) connected directly to the mixing chamber (5) allows the protein time to fold prior to adsorption onto the magnetic particles. Subsequently, a suspension of particles (8) was mixed with the stream, containing the folded protein, in another mixing chamber (10) and passed through a kinked pipe reactor (11). The second reactor (11) ensures close contact between the protein and the magnetic particles in order to form protein-particle complexes prior to capture in the filter canister (16).

**Wash and elution:** After capturing all of the protein-loaded particles in the filter canister (16), the recycling loop (14) was immediately filled with binding buffer (19) and closed using valves (12, 17, 18). Particles were released from the canister into the closed loop by switching off the external field and flushing the canister by pumping the enclosed liquid in the reversed direction when compared to loading. Physically entrained and/or loosely adsorbed contaminants were removed by recycling the particles in the closed loop at a flow velocity of 98 m/h for 600 s Washed particles were subsequently re-captured at 12 m/h in the filter canister by re-applying the magnetic field. Washings were pumped to the fraction collector (22), while at the same time filling the system with elution buffer (21). Cycles of releasing and washing/eluting and re-capturing the magnetic supports could be repeated until sufficient amounts of purified protein were recovered.

Recovery of the magnetic particles: Washed and desorbed particles were released from the canister by switching off the magnetic field and flushed out of the system by pumping binding buffer to the fraction collector.

Conditions for continuous refolding and product elution: Continuous folding was conducted at a constant total protein concentration of either 200 µg/ml or 100 µg/ml (in both cases the urea concentration was 267 mM) by dilution with binding buffer as described above and the protein was allowed 14 s in the pipe reactor to reach the correctly folded state under these conditions. Adsorption of the folded protein to the Cu²⁺-charged magnetic metal chelate adsorbents was performed at protein concentrations of 50 and 100 µg/ml, respectively, using a particle concentration of 3 mg/ml and a residence time in the inline pipe reactor of 10 s. Product-loaded supports were captured in the magnetic filter using a superficial linear flow velocity of 12 m/h and loading was stopped when breakthrough of the supports reached 10% of the concentration entering the filter. Supports were washed twice with binding buffer as described above. Soluble bound product was eluted with 500 mM imidazole in 500 mM NaCl, 20 mM Tris-HCl, pH 8 in 5 consecutive elution steps. Subsequently, insoluble protein was eluted in 3 steps with a denaturing elution buffer (elution buffer in 8 M urea) and disulphide cross-linked and aggregated protein was removed using the denaturing elution buffer supplemented with 25 mM 2-mercaptoethanol.

### Removal of the HAT-tag with Factor Xa

Folded and purified preparations of HAT-hβ₂M were cleaved with Factor Xa to release the native hβ₂M molecule. Eluted fractions were pooled and concentrated on a 3 kDa cut-off membrane (Millipore, USA) in a stirred ultrafiltration cell (Amicon, USA) at 5 °C. Concentrated solutions (~15 ml) were adjusted to 50 mM Tris-HCl, pH 8, 100 mM NaCl, 1 mM CaCl₂, 0.1 mM NiSO₄, 0.01% NaN₃ and the cleavage reaction was initiated by adding Factor Xa to a final concentration of 1 µg/ml. The cleavage reaction was essentially completed after incubation at room temperature for approximately 48 h. Conversion of HAT-hβ₂M to hβ₂M was monitored by SDS-PAGE analysis.

### Analytical Methods

### Protein analysis

The protein concentration was measured on a COBAS MIRA spectrophotometer (Roche, Switzerland) using the bicinchoninic acid (BCA) assay described by Smith *et al.,* (1985).

### Electrophoresis

One-dimensional sodium dodecyl sulphate-polyacrylamide gel electrophoresis was performed according to Laemmli (1970) using 1 mm pre-cast 4-12% NuPage SDS-PAGE gels. Gels were run on the XCell *SureLock^{™}* Mini-cell electrophoresis system with MES running buffer according to the manufacturer's instructions (Invitrogen, CA, USA). Samples were diluted in lithium-dodecyl sulphate sample buffer (Invitrogen, CA, USA) supplemented with either 4 mM iodoacetic acid or 1% (v/v) 2-ME to produce non-reducing and reducing conditions, respectively and a total volume of 10 µL was applied to the gel. Protein standards were Mark12^{™} (Invitrogen, CA, USA). Protein bands were stained with Coomassie Blue brilliant G-250. Densitometric analysis was performed on non-reducing SDS-polyacrylamide gels using the Quantity One^{®} Software package (Bio-rad Laboratories, USA). For SDS-PAGE analysis of the denatured and folded feedstock suspensions a total of 10 µg of protein was loaded on the gel unless otherwise stated. Protein samples from unbound pools as well as eluted and cleaned fractions were concentrated by acetone precipitation prior to SDS-PAGE analysis in the following way. Samples (100 µL) were mixed with cold acetone (-20 °C) in a ratio of 1:5 (v/v) before storage in the freezer (-20 °C) for at least 0.25 h. Precipitated protein was subsequently collected by centrifugation at 15,000 g for 0.1 h at 5 °C and re-dissolved in sample buffer (12 µL). A total volume of 10 µL was then loaded into each lane on the gel.

### Peptide - Major Histocompatibility Complex class I binding assay

The biological activity of the refolded and purified hβ₂M was tested essentially as described by Pedersen *et al.,* (1995). In short, varying concentrations of hβ₂M were added to a mixture of Major Histocompatibility Complex class I (MHC-I) heavy chain (truncated HLA-A*1101, 3 nM) and radiolabeled peptide (single letter code: KLFPPLYR, 1-3 nM) and incubated at 18°C for approximately 24 h to allow folding and complete maturation of the receptor complex. MHC-I heavy chains used in the assay were produced recombinantly by *E. coli* fermentations and purified to homogeneity as described by Ferré *et al.,* (2003). Folded MHC-I complexes, containing the radiolabeled peptide, were separated from unbound peptides by Sephadex G-50 spun column chromatography as described by Buus et *al.,* (1995). The radioactivity of the excluded 'void' volume containing the correctly folded complexes, and the retained volume, containing unbound peptides, was measured by gamma spectrometry (Packard Instruments, USA). Mean binding values were calculated by dividing excluded radioactivity of the duplicated samples with the total amount of radioactivity applied and these were plotted against varying amounts of hβ₂M.

### Results: Continuous refolding and adsorption onto Cu²⁺-charged IDA-linked magnetic adsorbents followed by separation in a high gradient magnetic separator

Continuous refolding and adsorption combined with direct separation, elution and collection of the product in a high gradient magnetic separator was conducted as described in Materials and methods (see Figure 2 for an overview of the system). A high gradient magnetic separator was employed to efficiently capture the tiny magnetic adsorbents (refer to Materials and methods for more details). Figure 3 shows SDS-PAGE analyses of fractions collected during elution and Table 2 presents the recoveries.

The eluted product was essentially 100% pure as judged by densitometric analysis of the SDS-polyacrylamide gel and the total recovery of folded monomeric HAT-hβ2m was approximately 50%. Higher recoveries could potentially have been obtained as the last of the eluted fraction still contained considerable amounts of monomeric HAT-hβ2m (see Figure 3, lane 7). To distinguish between adsorption/elution efficiency and folding recovery, the eluted folded HAT-hβ2m pool (Figure 3, lanes 3-7) was desalted, re-applied to the support and eluted as described above. The recovery of folded HAT-hβ2m from the support was 94%, demonstrating that the correctly folded molecule did not aggregate upon binding to the support. Rather, the loss of approximately 50% of the product is mainly the result of product misfolding during the batch folding by dilution reaction.

**Table 2. HAT-hβ2m recoveries after continuous refolding and adsorption onto Cu²⁺-charged magnetic supports combined with direct separation in a high gradient magnetic separator**

| Sample | Volume (mL) | Total Protein (µg) | Total HAT-hβ2M (µg) | Concentration factor (fold) | Total HAT-hβ2M recovery (%) | Purity^{a} (%) |
|---|---|---|---|---|---|---|
| Folded suspension^{a} | 200 | 13400 | 9045 | 1 | 100^{b} | 67.5 (±4.7)^{c} |
| Combined Elutions (0.5 M imidazole) | 60 | 4837 | ~4837 | 1.8 | 53 | ~100 |

| | | | | | | |
|---|---|---|---|---|---|---|
| Approximately 5% of the total protein was lost in the flow-through fraction. ^{a} Numbers are based on the total amount present after the 2-fold dilution in the adsorbent stream (see materials and methods) ^{b} Assuming a 100% refolding efficiency ^{c} Average of 4 independent densitometric analyses of non-reducing Coomassie stained gels of the refolded suspension is shown with standard deviations. | | | | | | |

For simplicity, we have used the simple folding by dilution reaction, and further optimisation may therefore significantly improve the overall recovery of the product. One obvious way of doing this would be to supplement the folding reaction with additives, such as detergents or redox pairs (e.g. GSH/GSSG), to minimize aggregation and promote disulphide bond formation.

The biological activity of the recovered molecule was tested in a peptide-MHC-I assay as described in Materials and method. Prior to analysis, the histidine-rich tail was removed by Factor Xa cleavage as it might influence the result of the biological assay, which is dependent on complex formation between hβ2m and the MHC class I heavy chain. The ability of the heavy chain part of the MHC-I receptor to fold and bind peptide is completely dependent on the presence of correctly folded β2m. In the absence of β2m, the heavy chain part misfolds and aggregates and a stable receptor complex capable of binding the presented peptide is not formed. The biological activity of the generated hβ2m molecule can therefore be evaluated in a folding reaction in which heavy chain, hβ2m and a radiolabeled peptide are added together. The amount of radiolabeled peptide bound to the receptor is a direct measure of the ability of the added hβ2m molecule to support folding of the heavy chain part of the receptor complex. The more peptide bound, the better the added hβ2m molecule supports folding of the heavy chain. Figure 4 shows the result from a dose response experiment in which graded doses of hβ2m have been added to a fixed amount of heavy chain and radiolabeled peptide together with an SDS-PAGE analysis of the HAT-hβ2m product before and after Factor Xa cleavage.

Peptide binding was observed in the region of 0.1 -10 nM, which is comparable or better than results obtained with hβ2m purified directly from ureamic patients or produced and purified from recombinant productions using *E. coli* (Pedersen *et al.,* (1995)). The quality of the product described is therefore of a very high standard even with the small pilot scale process employing the present invention.

### Example 2: Determination of operating conditions for continuous binding of HAT-hβ2m to magnetic adsorbents.

Here an example is given of how the operating conditions for one part of the process (namely the continuous binding of the assembled protein onto magnetic adsorbents) can be determined. First, it is outlined how the invention depicted in Example 1 can be used for determining the operating conditions for this step and a schematic of the system used is presented and described. Second, the binding capacity and then the binding kinetics of the adsorbents are determined in batch reactions. Then, it is demonstrated how to check that protein can be eluted from the adsorbents. Finally, it is shown how the information previously determined in this example can be used for designing the step of continuous adsorption of folded hβ2m onto the magnetic adsorbents followed by elution of the captured protein.

### Materials and Methods

HAT-hβ2m was prepared as described in Example 1

Essentially coated, non-porous superparamagnetic particles were prepared as in Example 1.

Analysis was performed as described in Example 1.

Size measurements of the prepared supports were conducted on a Mastersizer 2000 (Malvern, United Kingdom) according to manufacturer's instructions. Particles were resuspended in water and stirred at 3000 rpm on a Hydro2000SM mixer prior to analysis.

### Magnetic separation

The magnetic separator consisted of a test tube and a strong neodymium-iron-boron (Nd-Fe-B) permanent magnet block (Danfysik A/S, Jyllinge, Denmark), or a side pull rack (150-200 mT) (PerSeptive Biosystem, Framingham, Mass, USA).

### Determination of the binding characteristics of the adsorbents

Static batch binding studies were conducted with BSA and folded HAT-hβ₂M to estimate the capacity and dissociation constants of the prepared supports. IDA-linked supports were charged with 0.1 M CuSO₄ and equilibrated with binding buffer (0.5 mM NaCl in 20 mM Tris-HCl, pH 8). Equilibrated supports (3 mg) were incubated with BSA and folded HAT-hβ₂M, respectively, in the concentration range 0-2 mg/mL for 0.5 h at room temperature. Supernatants were subsequently assayed for unbound proteins using the BCA assay described in Materials and methods. All data sets were fitted to the simple Langmuir model (Langmuir, 1918) using the non-linear least squares fitting function in Origin 4.1 (Microcal software Inc., Northampton, USA) to estimate the maximum binding capacity (*Q*ₘₐₓ) and the dissociation constant (*K*_{d}).

Adsorption kinetics were determined by measuring the unbound protein fraction at different time points (20-1200 s) after mixing a constant amount of supports (1.5 mg or 3 mg) with folded HAT-hβ₂M (0.1 mg/mL or 2 mg/mL). Particles were settled on an Nd-Fe-B permanent magnet (~ 20 s) and the supernatant was immediately removed and assayed for protein content using the BCA assay (see Materials and methods). Data sets were fitted to a mass-transfer model described by Chase (1984). Rate constants for adsorption (k₁) and desorption (k₂) are determined from the estimated Qₘₐₓ and *K_{d}* (= *K₁*/*K₂)* values form the Langmuir fit of the equilibrium data using an empirical solution described by Sharma and Agarwal (2002).

### Batch folding of HAT-hβ2m

Batch folding (total volume of 10 ml) was carried out by diluting the denatured Feedstocks A and B in binding buffer (i.e. 20 mM Tris-HCl, pH 8, 500 mM NaCl). The final protein concentration was 200 µg/mL and the urea concentration was kept constant at 266 mM. A sample (1 mL) was removed for SDS-PAGE and BCA analysis. For SDS-PAGE analysis, a sample (100 µl) was immediately precipitated with cold acetone as described in Materials and methods to quench the folding reaction.

### Batch adsorption of folded HAT-hβ2m onto Cu²⁺-charaed magnetic chelators

Magnetic particles were equilibrated at the binding conditions and subsequently settled on the Nd-Fe-B permanent magnet to enable removal of the binding buffer. Respective batch folding mixtures (9 mL) were then added to the particles and incubated for 600 s on a rotating end mixer to bind the folded molecule to the supports. After particle settling the supernatants were collected and assayed for protein content as described previously.

### Continuous adsorption of batch folded HAT-hβ2M onto Cu²⁺-charged IDA-linked magnetic adsorbents

A modified part of the system depicted in Figure 3 was used for this example and this is depicted in Figure 5.

Continuous adsorption of already folded HAT-hβ₂M onto magnetic supports was investigated by concurrent mixing of two liquid streams, one containing the assembled protein of interest, and the other containing suspended adsorbents, in a pipe reactor (see Figure 5). The flow rates of the liquid streams were controlled by two peristaltic pumps (Amersham Biosciences, Sweden) which were carefully calibrated to give linear flow velocities of 150 m/h. IDA-linked adsorbents used for these experiments were charged and equilibrated in binding buffer as described in Example 1. Batch folding was done by dilution of the denatured feedstock in binding buffer (500 mM NaCl, 20 mM Tris-HCl, pH 8) to yield a final protein concentration of 200 µg/mL (266 mM urea, 200 mL scale). After pumping the two streams into a flow through 'F'-shaped mixer (inlets and outlet had an inner diameter of 1 mm) the final particle concentration was held constant at 3.0 mg/mL and the HAT-hβ₂M concentration was 100 µg/mL. The mixing chamber was directly connected to a pipe reactor with a variable length of 0 - 5 m, making it possible to measure different contact times (0 - 60 s) between the protein and particles. The pipe reactor used was made of hard teflon (i.d. 1 mm) tubing and equipped with kinks at every 50 mm to generate turbulent flow conditions during protein adsorption and to simulate a pipe reactor with a static mixer. After 300 s triplicate samples of HAT-hβ₂M-loaded particles were collected using the Nd-Fe-B magnet block separator. The supernatant was immediately withdrawn and analyzed for protein content by the BCA method and SDS-PAGE (see Example 1). Prior to elution, the collected particles were washed twice with binding buffer (500 mM NaCl in 20 mM Tris-HCl, pH 8) to remove unbound proteins. Bound proteins were eluted in batch mode by resuspending the collected particles in elution buffer (500 mM imidazole, 500 mM NaCl in 20 mM Tris-HCl, pH 8) followed by incubation with mixing for 600 s. Elution steps were repeated 5 times to remove all of the adsorbed proteins. Subsequently, the magnetic supports were cleaned under denaturing conditions (elution buffer in 8 M urea, repeated 3 times) followed by another cleaning step under denaturing and reducing conditions (elution buffer in 8 M urea and 25 mM 2-mercaptoehanol, repeated 3 times) to account for the aggregated and disulphide cross-linked protein. All washing, elution and cleaning steps were conducted with a total volume of 1 ml buffer.

### Results

### Determination of the binding strength and capacity of the magnetic adsorbents

Cu²⁺-charged superparamagnetic particles with an essentially non-porous matrix were prepared as described in materials and methods and the supports were subsequently characterized (i.e. in respect to size, capacity and binding kinetics) prior to use. Size measurements demonstrated that more than 90% of the particles had a diameter of less than 1.2 µm. Due to the tiny size of the magnetic particles and the thin outer coating, higher binding capacities and faster adsorption kinetics can be achieved as compared to conventional chromatographic supports. Maximum binding capacities (Qₘₐₓ) and dissociation constants (K_{d}) for adsorption of bovine serum albumin (BSA) and folded HAT-hβ2m to the prepared supports were determined and the results are summarised in Table 3 and Figure 6.

Figure 6 shows that the non-specific binding to the support under the conditions used is well below 5%, indicating that the adsorption is driven by the interaction between the Cu²⁺-ion of the target molecule. The initial slope (Qₘₐₓ *K*_{d}⁻¹) of the isotherms reflects the 'tightness' of binding to the support and is an essential parameter for particles, which are to be used in a continuous adsorption process, that is dependent on fast and strong binding kinetics. Adsorption capacities for BSA and HAT-hβ2m were in the same range (80-100 mg/g), whereas the K_{d} and the tightness of binding (given by Qₘₐₓ *K*_{d}⁻¹) was approximately 25-fold lower and 140 times higher, respectively, for HAT-hβ2m when compared to BSA. Binding of proteins to metal chelate adsorbents, such as the Cu²⁺-charged IDA-linked particles described herein, is dependent on the number of surface exposed histidines that are able to interact with the metal ion on the particle surface. In contrast to BSA, which only contains two exposed histidines, the HAT-hβ2m molecule has been genetically engineered to contain a histidine rich tail (i.e. 6 histidines placed non-adjacently to each other) and as a direct result of this, the interaction of the selected target molecule is much stronger than for the model molecule BSA.

**Table 3. Summary of the estimated Langmuir parameters^{a} (K_{d} and Qₘₐₓ) for adsorption of BSA and folded HAT-hβ2m to Cu²⁺ -charged IDA-linked magnetic supports**

| Support type | Target molecule | Qₘₐₓ (mg g⁻¹) | K_{d} (µM) | Qₘₐₓ K_{d}^{-1b} (L g⁻¹) |
|---|---|---|---|---|
| Cu²⁺-charged IDA-linked | BSA | 77.7 (±1.5) | 1.2 (±0.1) | 1 |
| Cu²⁺-charged IDA-linked | Folded HAT-hβ2m | 98.5 (±3.5) | 0.050 (±0.009) | 143 |

| | | | | |
|---|---|---|---|---|
| ^{a} Figure 6 adsorption data was fitted to the Langmuir model (well known to those skilled in the art) using the X²-minimisation procedure of Microcal Origin version 4.1. ^{b}Qₘₐₓ *K*_{d}⁻¹ reflects the tightness of the binding | | | | |

### Determination of the binding kinetics of the magnetic adsorbents

The time required for binding to the magnetic support is a crucial parameter, as it will directly affect the necessary length of the adsorption reactor. Binding kinetics for adsorption of folded HAT-hβ₂M to Cu²⁺-charged IDA-linked supports were therefore investigated at analytical scale in batch mode as described in the Materials and methods section. Results from this analysis are presented in Figure 7 and the data sets were fitted to the mass-transfer model described by Chase (1984). Rate constants for adsorption (k₁) and desorption (k₂) were estimated to 1.4 x 10³ M⁻¹s⁻¹ and 9.1 × 10⁻⁵ s⁻¹, respectively, using the empirical solution (Sharma and Agarwal (2002)) to the mass-transfer model. Values for Qₘₐₓ and K_{d} estimated from the Langmuir isotherms of folded HAT-hβ2m (see Table 3) were used in the model. The very low desorption value indicates that the protein is bound very tightly to the support.

Figure 7 demonstrates that fast adsorption kinetics can be obtained with these particles as equilibrium was reached in approximately 50 s with 70% of the protein bound to the support.

The results of the analysis described above simply illustrate that the magnetic support and the target protein can be designed in such a way that fast and strong binding kinetics are obtained. The possible combinations of support chemistries (i.e. base matrix, activation, coupling and fictionalization) and genetic constructs of the target proteins to assist separation are essentially unlimited and can be selected by the person skilled in the art. Standard methods for genetic manipulation and support preparation are described in the art.

### Demonstration of elution of HAT-hβ2M bound onto Cu²⁺-charged IDA-linked magnetic adsorbents

To demonstrate that the folded protein could be recovered after binding onto the magnetic adsorbents a simple demonstration using batch wise processing was employed. The elution efficiency of the Cu²⁺-charged IDA-linked support was evaluated after adsorption of batch folded HAT-hβ₂M. Protein refolded in a batch process was used with a total protein concentration of 200 µg/mL and a final urea concentration of 266 mM. The folded HAT-hβ₂M was subsequently mixed batch-wise with equilibrated magnetic supports. Soluble bound proteins were then eluted in batch mode and particles were afterwards cleaned with a denaturing buffer to account for the aggregated protein. A final cleaning step under denaturing and reducing conditions was included to remove any aggregated and disulphide cross-linked proteins. Figure 8 presents an SDS-PAGE analysis of collected fractions after elution and cleaning and Table 4 summarised the folding and adsorption/elution recovery of the product.

Prior to elution, the particles were washed with binding buffer to remove unbound proteins and the SDS-PAGE analysis of the resulting supernatant showed that no HAT-hβ2m was lost during this step (see Figure 8, lane 5), confirming that the binding to the support was very strong (see also Table 3) and that the amount of support used was sufficient to capture all of the product. Elution was performed in 5 consecutive steps and the total recovery (i.e. folding and elution recovery) of monomeric HAT-hβ2m was 52%. To distinguish between adsorption/elution efficiency and folding recovery, the eluted folded HAT-hβ2m pool (Figure 8, lanes 6-10) was desalted, re-applied to the support and eluted as described above. The recovery of folded HAT-hβ2m from the support was 94%, demonstrating that the correctly folded molecule did not aggregate upon binding to the support. Rather, the loss of 39% of the product is mainly the result of product misfolding during the batch folding reaction mediated by dilution. For simplicity, we have used the simple folding by dilution reaction and further optimisation may therefore significantly improve the overall recovery of the product. One obvious way of doing this would be to supplement the folding reaction with additives, such as detergents or redox pairs (e.g. glutathione and reduced glutathione), to minimize aggregation and promote disulphide bond formation.

Prior to elution, the particles were washed with binding buffer to remove unbound proteins and the SDS-PAGE analysis of the resulting supernatant showed that no HAT-hβ2m was lost during this step (see Figure 8, lane 5), confirming that the binding to the support was very strong (see also Table 3) and that the amount of support used was sufficient to capture all of the product. Elution was performed in 5 consecutive steps and the total recovery (i.e. folding and elution recovery) of monomeric HAT-hβ2m was 52%. To distinguish between adsorption/elution efficiency and folding recovery, the eluted folded HAT-hβ2m pool (Figure 8, lanes 6-10) was desalted, re-applied to the support and eluted as described above. The recovery of folded HAT-hβ2m from the support was 94%, demonstrating that the correctly folded molecule did not aggregate upon binding to the support. Rather, the loss of 48% of the product is mainly the result of product misfolding during the batch folding reaction mediated by dilution (see Table 4). For simplicity, we have used the simple folding by dilution reaction and further optimisation may therefore significantly improve the overall recovery of the product. One obvious way of doing this would be to supplement the folding reaction with additives, such as detergents or redox pairs (e.g. glutathione and reduced glutathione), to minimize aggregation and promote disulphide bond formation.

**Table 4. Summary of the performance of Cu²⁺-charged IDA-linked magnetic supports in batch mode**

| Sample | Volume (mL) | Total Protein (µg) | Total HAT- hβ2M (µg) | Folding recovery (%) | Adsorption/ Elution recovery ^{a} (%) | Total HAT-hβ₂M recovery (%) | Purity (%) |
|---|---|---|---|---|---|---|---|
| Folded suspension^{b} | 10 | 856 | 577.8 | 100 | 100 | 100 | 67.5 (±4.7)^{c} |
| Combined Elution (0.5 M imidazole) | 5 | 342 | 302 | 55.6 | 94 | 52.3 | 93 |

| | | | | | | | |
|---|---|---|---|---|---|---|---|
| ^{a} Value based on the total recovery (i.e. capture and elution) of correctly folded HAT-hβ2m as described in the text ^{b} Numbers are based on the total amount present after the 2-fold dilution in the adsorbent stream (see materials and methods) ^{c}Average of 4 independent densitometric analyses of non-reducing Coomassie stained gels of the refolded suspension is shown with standard deviations. | | | | | | | |

### Continuous adsorption of batch folded HAT-hβ2M onto Cu²⁺-charged magnetic adsorbents followed by elution of the bound protein

It is demonstrated below that continuous adsorption of the folded protein onto the magnetic adsorbents can be coupled with a means of recovering the protein of interest in solution, by releasing the bound product from the magnetic particles in a simple magnetic separator (see Figure 5).

Continuous adsorption of the batch folded HAT-hβ2m molecule onto the Cu²⁺-charged IDA-linked magnetic supports was tested by concurrently pumping a particle suspension and a batch folded protein suspension through a kinked mixing pipe reactor (See Figure 5). Refer to Materials and methods for a detailed description of the pipe reactor. Product-loaded magnetic particles were collected on a permanent magnet block separator after different retention times in the reactor and the resulting supernatants were assayed for unbound proteins as described in Materials and methods. Figure 9 shows the results from experiments conducted with a particle concentration of 3.0 mg/ml and a total protein concentration of 100 µg/ml during mixing.

Under the conditions selected above, essentially all of the folded HAT-hβ₂M molecules could be pulled out of solution using an inline statically mixed reactor with a 10 s retention time (Fig. 9). The rapid binding kinetics help to make the scaling of the process more simple as the retention time and thus the length of the continuous adsorption pipe reactor can be kept to a minimum.

The recovery of the process was then examined. Over a period of 150s, separate streams of HAT-hβ₂M and Cu⁺² charged IDA-linked magnetic adsorbents were pumped through the pipe reactor (with a 10 s residence time) and the loaded magnetic particles collected in a tube, using the permanent magnet as depicted in Figure 5. The adsorbed HAT-hβ₂M was eluted in batch mode as described in Materials and methods. Figure 10 shows the SDS-PAGE analysis of the collected fractions and Table 5 presents a summary of the data.

All of the absorbed target molecules were eluted in three steps (see Figure 10, lanes 5-7) and the purity was essentially 100% and the total recovery was 53% (see Table 5). The total recovery cannot be directly compared to that shown in Table 4 for the batch adsorption experiment as a different feedstock was used. Based on this analysis, it is evident that the manufactured particles were able to capture the folded product fast and very efficiently under continuous conditions and that the product could be recovered from the particles during the subsequent elution. The parameters determined here can subsequently be employed in the design of a process similar to that shown in Example 1.

**Table 5. Summary of recoveries obtained after continuous adsorption of batch folded HAT-hβ2m onto Cu²⁺-charged IDA-linked magnetic particles**

| Sample | Volume (mL) | Total protein (µg) | Total HAT-hβ2M (µg) | Concentration factor (fold) | Total HAT-hβ2M recovery (%) | Purity (%) |
|---|---|---|---|---|---|---|
| Folded suspension^{a} | 10 | 1073 | 724 | 1 | 100^{b} | 67.5 (±4.7)^{c} |
| Combined Elutions (0.5 M imidazole) | 5 | 388 | 388 | 1.1 | 53 | 98.6 |
| Combined CIP (8 M Urea) | 3 | 188 | 179 | 0.8 | 25 | 95.3 |

| | | | | | | |
|---|---|---|---|---|---|---|
| CIP = Clean-In-Place ^{a} Numbers are based on the total amount present after the 2-fold dilution in the adsorbent stream (see materials and methods) ^{b} Assuming a 100% refolding efficiency ^{c} Average of 4 independent densitometric analyses of non-reducing Coomassie stained gels of the refolded suspension is shown with standard deviations. | | | | | | |

### Example 3: Continuous refolding and adsorption of HAT-hβ₂M onto Cu²⁺-charged magnetic metal chelate adsorbents

This is an example of how the operating conditions for one part of the process (namely the continuous refolding of the protein followed by its adsorption onto magnetic adsorbents) can be determined. First, a variation of the invention shown in Example 1 is presented and described which is suitable for determining the continuous refolding and adsorption conditions, and its employment is described using a concrete example.

### Materials and Methods

HAT-hβ2m was prepared as described in Example 1.

Essentially coated, non-porous superparamagnetic particles were prepared as in Example 1.

Analysis was performed as described in Example 1.

Magnetic separation was performed as described in Example 2.

A modified version of the system used in Example 1 (see Figure 2) was employed in this example and is schematically represented in Figure 11.

### Results

### Continuous refolding combined with continuous adsorption onto the Cu²⁺-charged IDA-linked magnetic supports

Continuous folding and adsorption onto the magnetic supports was coupled to adsorbent capture using a permanent magnet block separator as shown in Figure 11. Folding of HAT-hβ2m was initiated by dilution of the denatured preparation (i.e. Feedstock B) with binding buffer in the first mixer. The flow rate of the pumps controlling the speed at which Feedstock B and binding buffer entered the mixer was adjusted in such way that the protein concentration during folding was kept constant at 200 µg/ml and the urea concentration was 266 mM. Based on the fast folding kinetics of the hβ2m protein (Chiti *et al.,* 2001), the length of the folding reactor was adjusted to give a retention time of only 14 s. Folded HAT-hβ2m was directly captured onto Cu²⁺-charged IDA-linked magnetic adsorbents by mixing the particle suspension with the protein suspension in another mixer (see Figure 11). The flow rate at which the particle suspension was introduced into the protein stream was adjusted to give conditions for adsorption of 3 mg particles/ml and 100 µg total protein/ml. Formation of product-particle complexes was done under turbulent conditions for at total period of 10 s as described above. The whole process was run for 150 s and during this time product-loaded particles were collected after which the HAT-hβ₂M was recovered as described above. Fractions collected during batch elution were analysed for protein content by SDS-PAGE (see Figure 12). A summary of the recoveries during the process is presented in Table 6.

The SDS-PAGE analysis demonstrated that all of the folded product was captured on the support, as no soluble protein could be detected in the supernatant after particle collection (see Figure 12, lane 5). Furthermore, the product was eluted in essentially pure form (see Figure 12, lanes 6-10), as was the case in Example 2. The retention time of 14 s did not significantly affect the folding efficiency as the total recovery of HAT-hβ₂M was similarly to what was reported for the batch folding experiment (see Example 2) (i.e. folding for 0.5 h) coupled with continuous capture (compare recoveries in Tables 5 and 6). In general, the continuous refolding and adsorption process produced slightly higher recoveries showing that both elements (i.e. folding and adsorption) could be run in continuous mode without a detrimental effect on performance.

**Table 6. Summary of recoveries after continuous refolding and adsorption of HAT-hβ2m onto Cu²⁺-charged IDA-linked supports**

| Sample | Volume (mL) | Total protein (µg) | Total HAT-hβ₂M (µg) | Concentration factor (fold) | Total HAT-hβ₂M recovery (%) | Purity (%) |
|---|---|---|---|---|---|---|
| Folded suspension^{a} | 10 | 1219 | 823 | 1 | 100^{b} | 67.5 (±4.7)^{c} |
| Combined Elutions (0.5 M imidazole) | 5 | 486 | ~486 | 1.2 | 59 | ~100 |

| | | | | | | |
|---|---|---|---|---|---|---|
| ^{a} Numbers are based on the total amount present after the 2-fold dilution in the adsorbent stream (see materials and methods) ^{b} Assuming a 100% refolding efficiency ^{c} Average of 4 independent densitometric analyses of non-reducing Coomassie stained gels of the refolded suspension is shown with standard deviations. | | | | | | |

### Example 4: Effect of modification of fluid flow conditions in the continuous pipe reactor on adsorption of HAT-hβ2m to Cu²⁺-charged magnetic chelate adsorbents.

This example has been included to illustrate the flexibility of the continuous adsorption step of the present invention. It is evident that turbulent conditions will create the fastest adsorption process possible, however, in the case of a shear sensitive protein or macromolecular complex, such conditions might not be beneficial. We therefore tested the adsorption kinetics of batch folded HAT-hβ2m to the prepared supports under laminar flow conditions.

### Materials and methods

Refer to descriptions under Example 2. The layout of the system used for the process was similar to that of Example 2, except that the inline reactor consisted of a linear, variable length silicone tube (i.d. 1 mm).

### Results

Product-loaded particles were collected after different retention times in the pipe reactor and the resulting supernatants were analysed for protein content as described in Materials and methods (see Example 2). Conditions (i.e. particle concentration, protein concentration and flow rates) were identical to the turbulent adsorption experiment described in Example 2. The results are presented in Figure 13.

As expected, the time required to adsorb all of the folded HAT-hβ2m molecules was increased by 6-fold (from 10 s to 60 s) as compared to the turbulent mixing case (compare Figure 9 and 13). However, the time frame was still within that which is compatible with much larger process scales than those presented herein and the laminar conditions therefore did not compromise the ability to scale-up the process. Furthermore, the loss in adsorption speed may be compensated for by increasing the concentration of particles during binding.

### Example 5: Compatibility with solids containing liquids

The following example has been included to demonstrate the performance of the present invention when faced with the task of processing liquids containing insoluble by-products. In this example, it has specifically been chosen to conduct a batch refolding process that generates insoluble protein aggregate by-products and a turbid solution.

### Materials and Methods

Refer to Example 2, except with modifications to the batch refolding process as discussed below.

### Results

Formation of insoluble by-products, such as protein aggregates, is frequently associated with assembly reactions of the nature described above. To model such a system, a batch refolding reaction of HAT-hβ2m with 800 µg total protein/ml and a total NaCl concentration of 150 mM was used. These conditions resulted in a batch suspension where approximately 40% (C/C₀ = C_{insoluble}/C_{soluble} = 0.57 as estimated by the BCA assay (see Example 1)) of the protein came out of solution as insoluble protein aggregates. The hazy protein suspension resulting was continuously mixed with the Cu²⁺-charged IDA-linked magnetic support under turbulent conditions as described in Examples 1 and 2. Conditions for adsorption were 12 mg particles/ml at a total protein concentration of 400 µg/ml, resulting in the same particle/protein ratio as applied in the previous examples. HAT-hβ₂M-loaded particles were collected for 150 s and bound proteins were eluted. Collected fractions were analysed by SDS-PAGE (see Figure 14). Table 6 presents a summary of the recoveries obtained.

The unbound fraction did not contain any soluble proteins (see Figure 14A, lane 5), demonstrating the ability of the particles to effectively adsorb all of the soluble HAT-hβ₂M molecules from a suspension heavily contaminated with insoluble aggregates. As a result of the poor batch folding efficiency (Note! aggregates were deliberately forced to form in order to test this process) the total HAT-hβ2m recovery was reduced to 22% as compared to the total amount of protein in the system. Cleaning of the particles with denaturing and reducing buffers, accounted for the missing protein, which was bound to the support in an aggregated and disulphide cross-linked state (see Figure 14, lanes 5-9). This example clearly shows that the particles are compatible with suspensions containing particulate matter, such as protein aggregates.

**Table 7. Summary of recoveries from continuous adsorption of batch folded HAT-hβ2m from a suspension contaminated with protein aggregates onto the Cu²⁺-charged IDA-linked supports.**

| Sample | Volume (mL) | Total soluble protein (µg) | Total soluble HAT-hβ2M (µg) | Total HAT-hβ2M recovery (%) | Purity (%) |
|---|---|---|---|---|---|
| Folded suspension^{a} Combined elutions | 10 | 2265 | 1529 | 100^{b} | 67.5 (±4.7)^{c} |
| (0.5 M imidazole) Combined urea CIP | 5 | 340 | 336 | 22^{d} | 98.7 |
| (8 M Urea) Combined urea/reducing CIP | 3 | 1283 | 1227 | 80^{d} | 95.6 |
| (8 M Urea/25 mM 2-ME) | 3 | n.d.^{e} | n.d.^{e} | n.d.^{e} | 80.5 |

| | | | | | |
|---|---|---|---|---|---|
| n.d. = not determined; CIP = Clean-In-Place; 2-ME = 2 - mercaptoethanol; Total amount of protein applied to the system was 4000 µg, corresponding to 5 mL of a 800 µg/mL suspension. ^{a} Numbers are based on the total amount present after the 2-fold dilution in the adsorbent stream (see materials and methods) ^{b} Assuming a 100% refolding efficiency ^{c} Average of 4 independent densitometric analyses of non-reducing Coomassie stained gels of the refolded suspension is shown with standard deviations. ^{d} Calculated from the total amount of soluble monomeric HAT-hβ2m that enters the system, i.e. 1529 µg *^{e}* The total protein concentration could not be determined due to the presense of reducing agent, which interferes with the BCA assay. | | | | | |

### REFERENCES

Burton, S. C., and Harding, D. R. K. (1997): High-density ligand attachment to bromated allyl matrices and application to mixed mode chromatography of chymosin. J. Chromatogr. A. 775: 39-50.
Buus, S. Ferré, H. & Ruffet, E. (2001): A method for refolding of proteins. PCT application, Patent no. WO 02/057296.
Buus, S., Stryhn, A., Winther, K., Kirkby, N., and Pedersen, L. 0. (1995): Receptor-ligand interactions measured by improved spun column chromatography technique A high efficiency and high throughput size separation method. Biochim. Biophys. Acta. 1243: 453-460.
Carew, E.B. (1992): Method for separating pathogenic or toxic agents from a body fluid and return to body. United States Patent. Patent no. 5,123,901.
Chase, H. A. (1984): Prediction of the performance of preparative affinity chromatography. J. Chromatogr. 297 :179-202.
Chiti, F., Manginone, P., Andreola, A., Giorgetti, S., Stefani, M., Dobson, C. M., Bellotti, V., and Taddei N. (2001): Detection of two partially structured species in the folding process of the amyloidogenic protein β2-microglobuline. J. Mol. Biol. 307: 379-391.
Ferré, H., Ruffet, E., Blicher, T., Sylvester-Hvid, C., Nielsen, L. B., Hobley, T. J., Thomas, O. R. T., and Buus, S. (2003): Purification of correctly oxidized MHC class I heavy-chain molecules under denaturing conditions: A novel strategy exploiting disulfide assisted protein folding. Protein Sci. 3: 551-559.
Heeboll-Nielsen, A. (2002): High Gradient Magnetic Fishing. Support functionalisation and application for protein recovery from unclarified bioprocess liquors. Ph.D. Thesis, Technical University of Denmark, Lyngby, Denmark.
Hubbuch, J.J., (2000): Development of adsorptive separation systems for recovery of protein from crude bioprocess liquors' ph. D. thesis published by BioCentrum-DTU, Lyngby, Denmark.
Hubbuch, J.J., Matthiesen, D.B., Hobley, T.J. & Thomas, O.R.T. (2001): High gradient magnetic separation versus expanded bed adsorption: a first principle comparison. Bioseparation 10: 99-112.
Hubbuch, J.J. & Thomas, O.R.T. (2002): High-Gradient Magnetic Affinity Separation of Trypsin from Porcine Pancreatin. Biotechnol. Bioeng. 79: 301-313.
Laemmli, U.K. (1970): Clevage of structural proteins during assembly of the head of bacteriophage T4. Nature. 227: 680-685.
Langmuir, I. (1918): The adsorption of gases on plane surface of glass, mica and platinum. J. Am. Chem. Soc. 40: 1361-1403.
O'Brien, S.M., Thomas, O. R. T., and Dunhill P. (1996): Non-porous magnetic chelator supports for protein recovery by immobilised metal affinity adsorption. J. Biotechnol 50: 13-25.
Pedersen, L. Ø., Stryhn, A., Holtet, T. L, Etzerodt, M., Gerwein, J., Nissen, M. H., Thøgersen, H. C., and Buus, S. (1995): The interaction of beta 2-microglobuline (β2-m) with mouse class I major histocompatibility antigens and its ability to support peptide binding. Eur. J. Immunol.' 6: 1609-1616.
Smith, P. K., Krohn, R. I., Hermanson, G. T., Mallia, A. K., Gartner, F. H., Provenzano, M. D., Fujimoto, E. K., Goeke, N. M, Olson, B. J., and Klenk, D. C. (1985): 'Measurement of protein using the bicinchonic acid. Anal. Biochem. 150: 76-85.
Sharma, S., and Agarwal, G. P. (2002): Adsorption equilibrium and kinetics of egg-white proteins on immobilized metal ion affinity gels for designing fractionation. Adsorption. 8: 203-213.
Zulqurnain, K. (1999): Scale-up of affinity based separation based on magnetic support particles. Ph.D. thesis published by University College London.

## Claims

1. A continuous process for obtaining a preparation of a macromolecular assembly of one or more macromolecular substances including at least one substance selected from the group consisting of proteins, carbohydrates, nucleic acids, nucleic acid analogues, and protein-nucleic acid chimera, said process comprising the steps of:
a) (i) providing one or more fluid compositions each comprising at least one of said one or more macromolecular substances, said one or more macromolecular substances being in a predominantly unassembled form, (ii) providing a dispersion comprising coated, essentially non-porous magnetic particles, and (iii) optionally providing one or more additives;
b) simultaneously or sequentially combining said one or more fluid compositions, the dispersion of magnetic particles, and said one or more optional additives so as to provide a continuous stream com prising said one or more m acrom olecular substances, the magnetic particles and any optional additives;
c) passing said continuous stream through a first mixing device so as to form magnetic complexes each comprising a magnetic particle and a plurality of macromolecular assembly species;
d) passing the continuous stream through a first capture compartment of a magnetic separator so as to capture said magnetic complexes; and
e) separating said magnetic complexes from said continuous stream, and isolating said macromolecular assembly species from said magnetic particles so as to obtain the preparation of a macromolecular assembly of the one or more macromolecular substances.

2. The process according to claim 1, wherein said one or more macromolecular substances are selected from proteins.

3. The process according to claim 1, wherein said one or more macromolecular substances are selected from nucleic acids, nucleic acid analogues, and protein-nucleic acid chimera.

4. The process according to any one of the preceding claims, wherein the molecular weight of each of said one or more macromolecular substances is at least 1000 Da.

5. The process according to any one of the preceding claims, wherein only one macromolecular substance is required for the formation of the macromolecular assembly.

6. The process according to claim 5, wherein the macromolecular substance is represented by a single protein, the unassembled form of said protein being the unfolded, misfolded or aggregated form, and the molecular assembly of said protein being the biologically active, folded form.

7. The process according to any one of the claims 1-5, wherein at least two macromolecular substances are required for the formation of the macromolecular assembly.

8. The process according to claim 7, wherein said at least two macromolecular substances are represented by a plurality of proteins, the unassembled form of said proteins being the respective proteins in unfolded, misfolded or aggregated form, and the macromolecular assembly of said proteins being a biologically active, quaternary structure wherein the respective proteins represent subunits or domains.

9. The process according to claim 8, wherein the at least two macromolecular substances are represented by a plurality of species selected from nucleic acids, analogues of nucleic acids, and protein-nucleic acid chimera, the unassembled form of said substances being the non-hybridized forms of said species, and the molecular assembly of said substances being the mutually hybridized form of said species.

10. The process according to any one of the preceding claims, wherein the magnetic particles are superparamagnetic.

11. The process according to any one of the preceding claims, wherein magnetic particles carry one or more types of ligands.

12. The process according to claim 11, wherein the selected ligand(s) has/have affinity for at least one of said one or more macromolecular substances.

13. The process according to claim 11, wherein the selected ligand(s) has/have affinity for the macromolecular assembly.

14. The process according to claim 13, wherein the selected ligand(s) has/have substantially no affinity for the unassembled, individual macromolecular substances.

15. The process according to any one of the preceding claims, wherein, in step (b), said one or more fluid compositions and said one or more optional additives are combined and passed through a second mixing device so as to form a continuous pre-stream of a macromolecular assembly of the one or more macromolecular substances, said pre-stream subsequently being combined with the dispersion of magnetic particles so as to form the continuous stream referred to in step (b).

16. The process according to any one of the claims 1-14, wherein, in step (b), at least one of said one or more fluid compositions and the dispersion of magnetic particles are combined and passed through a second mixing device so as to form a continuous pre-stream of magnetic complexes of magnetic substances and one or more macromolecular substances, said pre-stream subsequently being combined with any remaining of said one or more fluid compositions and said one or more optional additives so as to form the continuous stream referred to in step (b).

17. The process according to any one of the claims 1-14, wherein, in step (b), said one or more fluid compositions, the dispersion of magnetic particles, and one or more optional additives are combined substantially simultaneously so as to form the continuous stream referred to in step (b).

18. The process according to any one of the preceding claims, wherein the first mixing device facilitates turbulent mixing conditions for the constituents of said stream.

19. The process according to any one of the preceding claims, wherein the magnetic separator has at least two operational modes, a first of said at least two operational modes providing a magnetic field to the first capture compartment thereby rendering the first capture compartment capable of capturing the magnetic complexes, and a second of said at least two operational modes providing an inadequate magnetic field to the first capture compartment thereby rendering the first capture compartment substantially incapable of capturing magnetic complexes.

20. The process according to any one of the preceding claims, wherein the magnetic complexes are separated from the continuous stream by redirecting said continuous stream to a second capture compartment.

21. The process according to claim 20, wherein the second capture compartment is a second capture compartment of the magnetic separator referred to in claim 1.

22. The process according to any one of the claims 1-19, wherein the magnetic complexes are separated from the continuous stream by interrupting said stream.

23. The process according to any one of the preceding claims, wherein at least a fraction of the continuous stream, after passage through the capture compartment of the magnetic separator, is continuously fed back upstream so as to become a part of the continuous stream of step (b).

24. The process according to any one of the preceding claims, wherein the magnetic particles are fed back upstream and mixed with the dispersion of magnetic particles or fed upstream to any of the mixers (10,11,5,6), after isolation of the macromolecular assembly.

25. A system useful for the continuous assembly and capture of macromolecular substances, the system comprising: a plurality of containers including at least one first container containing a dispersion of coated, essentially non-porous magnetic particles (8), at least one second container containing a liquid composition comprising one or more macromolecular substances (1), and optionally at least one third container (2) containing one or more additives; at least one pump (3,4,7) for causing the content of each of said containers (1,2,8) to be fed to a first mixing device (10,11); a conduit for providing passage from said first mixing device (10,11) to the first capture compartment (16) of a magnetic separator (15), said magnetic separator (15) having at least two operational modes, a first of the at least two operational modes providing a magnetic field to the first capture compartment (16) thereby rendering the first capture compartment (16) capable of capturing the magnetic particles (8), and a second of the at least two operational modes providing an inadequate magnetic field to the first capture compartment (16) thereby rendering the first capture compartment (16) substantially incapable of capturing the magnetic particles (8).

26. The system according to claim 25, wherein the first mixing device (10,11) comprises at least one first pipe reactor (11) for facilitating turbulent mixing conditions for the content of said containers (1,2,8).

27. The system according to any one of the claims 25-26, further comprising a second mixing device (5,6) arranged downstream relative to said at least one second container containing said one or more macromolecular substances (1 ) and said at least one third container containing one or more additives (2), and upstream relative to said first mixing device.

28. The system according to claim 27, wherein said second mixing device (5,6) comprises at least one second pipe reactor (6) facilitating turbulent mixing conditions for the combined content of said second and third containers fed thereto.

29. The system according to any one of the claims 25-28, further comprising a second mixing device (5,6) arranged downstream relative to said at least one first container containing the dispersed magnetic particles (8) and said at least one second container containing said one or more macromolecular substances (1), and upstream relative to said first mixing device.

30. The system according to claim 29, wherein said second mixing device (5,6) comprises at least one second pipe reactor (6) facilitating turbulent mixing conditions for the combined content of said second and third containers fed thereto.

31. The system according to any of claim 25-30, further comprising at least one recycle conduit (23,24,25) for providing a passage from the capture compartment (16) to a system member upstream relative to the first mixing device (10,11).

## Patentansprüche

1. Kontinuierliches Verfahren zum Erhalt einer Zubereitung einer makromolekularen Anordnung einer makromolekularen Substanz oder mehrerer makromolekularer Substanzen, die wenigstens eine Substanz enthält, die aus der Gruppe bestehend aus Proteinen, Kohlenhydrate, Nukleinsäuren, Nukleinsäurenanaloga und Proteinnukleinsäurechimären ausgewählt ist, wobei das Verfahren die Schritte aufweist:
a) (i) Bereitstellen einer Flüssigkeitszusammensetzung oder mehrerer Flüssigkeitszusammensetzungen, von denen jede wenigstens eine makromolekulare Substanz oder mehrere der makromolekularen Substanzen enthält, wobei die eine makromolekulare Substanz oder die mehreren makromolekularen Substanzen überwiegend in einer nicht zusammengefügten Form vorliegen, (ii) Bereitstellen einer Dispersion mit beschichteten, im Wesentlichen nicht porösen magnetischen Partikeln und (iii) optional das Bereitstellen eines oder mehrere Additive,
b) gleichzeitiges oder aufeinanderfolgendes Kombinieren der einen Flüssigkeitszusammensetzung oder der mehreren Flüssigkeitszusammensetzungen, der Dispersion an magnetischen Partikeln und dem einen optionalen Additiv oder den mehreren optionalen Additiven, um einen kontinuierlichen Strom bereitzustellen, der die eine makromolekulare Substanz oder die mehreren makromolekularen Substanzen, die magnetischen Partikel und jegliche optionale Additive aufweist,
c) Durchleiten des kontinuierlichen Stromes durch eine erste Mischvorrichtung, um magnetische Komplexe zu bilden, die jeweils ein magnetisches Partikel oder eine Anzahl von makromolekularen Zusammenbauspezies aufweist,
d) Durchleiten des kontinuierlichen Stromes durch eine erste Auffangabteilung eines magnetischen Separators, um die magnetischen Komplexe einzufangen,
e) Trennen der magnetischen Komplexe von dem kontinuierlichen Strom und Isolieren der makromolekularen Zusammenbauspezies von den magnetischen Partikeln, um die Zubereitung einer makromolekularen Anordnung der einen makromolekularen Substanz oder der mehreren makromolekularen Substanzen zu erhalten.

2. Verfahren nach Anspruch 1, bei dem die eine makromolekulare Substanz oder die mehreren makromolekularen Substanzen aus Proteinen ausgewählt sind.

3. Verfahren nach Anspruch 1, wobei die eine makromolekulare Substanz oder die mehreren makromolekularen Substanzen aus Nukleinsäuren, Nukleinsäurenanaloga und Proteinnukleinsäurechimären ausgewählt sind.

4. Verfahren nach einem der vorangehenden Ansprüche, bei dem das Molekulargewicht jeder der einen oder der mehreren makromolekularen Substanzen wenigstens 1000 Da ist.

5. Verfahren nach einem der vorangehenden Ansprüche, wobei lediglich eine makromolekulare Substanz zum Bilden der makromolekularen Anordnung erforderlich ist.

6. Verfahren nach Anspruch 5, wobei die makromolekulare Substanz durch ein einzelnes Protein dargestellt ist, wobei die nicht zusammengefügte Anordnung des Proteins in der nicht gefalteten, fehlgefalteten oder aggregierten Form vorliegt und wobei die molekulare Anordnung dieses Proteins in der biologisch aktiven gefalteten Form vorliegt.

7. Verfahren nach einem der Ansprüche 1 - 5, wobei wenigstens zwei makromolekulare Substanzen zum Ausbilden der makromolekularen Anordnung erforderlich sind.

8. Verfahren nach Anspruch 7, wobei die wenigstens zwei makromolekularen Substanzen durch eine Anzahl von Proteinen dargestellt sind, wobei die nicht zusammengefügte Form der Proteine die jeweiligen Proteine in nicht gefalteter, fehlgefalteter oder aggregierter Form ist und wobei die makromolekulare Anordnung der Proteine eine biologisch aktive quartäre Struktur ist, in der die jeweiligen Proteine Untereinheiten oder Domains darstellen.

9. Verfahren nach Anspruch 8, bei dem die wenigstens zwei makromolekularen Substanzen durch eine Anzahl von Spezies dargestellt sind, die aus Nukleinsäuren, Nukleinsäurenanaloga und Proteinnukleinsäurechimären ausgewählt sind, wobei die nicht zusammengefügte Form der Substanzen die nicht hybridisierten Formen der Spezies sind und wobei die molekulare Anordnung der Substanzen die wechselseitig hybridisierten Formen der Spezies sind.

10. Verfahren nach einem der vorangehenden Ansprüche, wobei die magnetischen Partikel superparamagnetisch sind.

11. Verfahren nach einem der vorangehenden Ansprüche, wobei die magnetischen Partikel einen Ligand oder mehrere Typen von Liganden tragen.

12. Verfahren nach Anspruch 11, wobei der ausgewählte Ligand beziehungsweise die ausgewählten Liganden eine Affinität für die eine makromolekulare Substanz oder mehrere der makromolekularen Substanzen aufweist beziehungsweise aufweisen.

13. Verfahren nach Anspruch 11, wobei der ausgewählte Ligand beziehungsweise die ausgewählten Liganden eine Affinität für die makromolekulare Anordnung aufweist beziehungsweise aufweisen.

14. Verfahren nach Anspruch 13, wobei der ausgewählte Ligand beziehungsweise die ausgewählten Liganden im Wesentlichen keine Affinität für die nicht zusammengefügten, einzelnen makromolekularen Substanzen hat beziehungsweise haben.

15. Verfahren nach einem der vorangehenden Ansprüche, wobei in dem Schritt (b) die eine Flüssigkeitszusammensetzung oder die mehreren Flüssigkeitszusammensetzungen sowie das eine optionale Additiv oder die mehreren optionalen Additive kombiniert werden und eine zweite Mischvorrichtung durchlaufen, um einen kontinuierlichen Vorstrom einer makromolekularen Anordnung der einen makromolekularen Substanz oder der mehreren makromolekularen Substanzen zu bilden, wobei der Vorstrom nach und nach mit der Dispersion an magnetischen Partikeln kombiniert wird, um den kontinuierlichen Strom gemäß Schritt (b) zu bilden.

16. Verfahren nach einem der Ansprüche 1 - 14, wobei in dem Schritt (b) wenigstens die eine Flüssigkeitszusammensetzung oder mehrere Flüssigkeitszusammensetzungen und die Dispersion an magnetischen Partikeln kombiniert werden und eine zweite Mischvorrichtung durchlaufen, um einen kontinuierlichen Vorstrom eines magnetischen Komplexes aus magnetischen Substanzen und der einen makromolekularen Substanz oder der mehreren makromolekularen Substanzen zu bilden, wobei der Vorstrom nach und nach mit einem beliebigen Rest der einen Flüssigkeitszusammensetzung oder der mehreren Flüssigkeitszusammensetzungen und dem einen optionalen Additiv oder den mehreren optionalen Additiven kombiniert wird, um den kontinuierlichen Strom gemäß Schritt (b) zu bilden.

17. Verfahren nach einem der Ansprüche 1 - 14, wobei in dem Schritt (b) die eine Flüssigkeitszusammensetzung oder die mehreren flüssigen Zusammensetzungen, die Dispersion an magnetischen Partikeln und ein optionales Additiv oder mehrere optionale Additive im Wesentlichen gleichzeitig kombiniert werden, um den kontinuierlichen Strom gemäß Schritt (b) zu bilden.

18. Verfahren nach einem der vorangehenden Ansprüche, wobei die erste Mischvorrichtung turbulente Mischbedingungen für die Bestandteile des Stromes fördert.

19. Verfahren nach einem der vorangehenden Ansprüche, wobei der magnetische Separator wenigstens zwei Betriebsarten aufweist, wobei ein erster der wenigstens zwei Betriebsarten ein magnetisches Feld in der ersten Auffangabteilung bereitstellt, wobei die erste Auffangabteilung **dadurch** dazu eingerichtet ist, magnetische Komplexe aufzufangen, und wobei ein zweiter der wenigstens zwei Betriebsarten ein nicht angepasstes Magnetfeld in der ersten Auffangabteilung bereitstellt, so dass **dadurch** die erste Auffangabteilung im Wesentlichen nicht dazu eingerichtet ist, magnetische Komplexe aufzufangen.

20. Verfahren nach einem der vorangehenden Ansprüche, wobei die magnetischen Komplexe aus dem kontinuierlichen Strom durch Umlenken des kontinuierlichen Stromes in eine zweite Auffangabteilung getrennt werden.

21. Verfahren nach Anspruch 20, wobei die zweite Auffangabteilung eine zweite Auffangabteilung des magnetischen Separators gemäß Anspruch 1 ist.

22. Verfahren nach einem der Ansprüche 1 bis 19, wobei die magnetischen Komplexe aus dem kontinuierlichen Strom durch Unterbrechen des Stromes separiert werden.

23. Verfahren nach einem der vorangehenden Ansprüche, wobei wenigstens ein Teil des kontinuierlichen Stromes nach Durchlaufen durch die Auffangabteilung des magnetischen Separators kontinuierlich in Strömungsrichtung rückgeführt wird, um ein Teil des kontinuierlichen Stromes von Schritt (b) zu werden.

24. Verfahren nach einem der vorangehenden Ansprüche, wobei die magnetischen Partikel in Strömungsrichtung rückgeführt und mit der Dispersion an magnetischen Partikeln gemischt oder in Strömungsrichtung einem beliebigen der Mischer (10, 11, 5, 6) nach Isolierung der makromolekularen Anordnung eingespeist wird.

25. System, das für den kontinuierlichen Zusammenbau und das Auffangen von makromolekularen Substanzen zweckmäßig ist, wobei das System aufweist: eine Anzahl von Behältern, die wenigstens einen ersten Behälter aufweist, der eine Dispersion an beschichteten, im Wesentlichen nicht porösen magnetischen Partikeln (8) beinhaltet, über wenigstens einen zweiten Behälter verfügt, der eine Flüssigkeitszusammensetzung beinhaltet, die eine makromolekulare Substanz oder mehrere makromolekulare Substanzen (1) aufweist, und optional mit einem dritten Behälter (2) ausgestattet ist, der ein Additiv oder mehrere Additive aufweist, wenigstens eine Pumpe (3, 4, 7) zum Fördern des Inhalts jedes Behälters (1, 2, 8) zu einer ersten Mischvorrichtung (10, 11), eine Leitung zum Bereitstellen eines Durchlasses von der ersten Mischvorrichtung (10, 11) zu der ersten Auffangabteilung (16) eines magnetischen Separators (15), wobei der magnetische Separator (15) wenigstens zwei Betriebsarten aufweist, wobei eine erste der beiden Betriebsarten in der ersten Auffangabteilung (16) ein magnetisches Feld bereitstellt, das die erste Auffangabteilung (16) dazu einrichtet, die magnetischen Partikel (8) einzufangen, und wobei eine zweite der beiden Betriebsarten der ersten Auffangabteilung (16) ein nichtangepasstes Magnetfeld bereitstellt, was die erste Auffangabteilung (16) dazu bringt, für ein Auffangen von magnetischen Partikeln (8) nicht angepasst zu sein.

26. System nach Anspruch 25, wobei die erste Mischvorrichtung (10, 11) wenigstens einen ersten Rohrreaktor (11) zum Fördern von turbulenten Mischungsbedingungen für den Inhalt der Behälter (1, 2, 8) aufweist.

27. System nach einem der Ansprüche 25 - 26, das weiterhin eine zweite Mischvorrichtung (5, 6) aufweist, die in Bezug auf den wenigstens einen zweiten Behälter, der die eine makromolekulare Substanz oder die mehreren makromolekularen Substanzen (1) aufweist, und den wenigstens einen dritten Behälter, der das eine Additiv oder die mehreren Additive (2) aufweist, in Strömungsrichtung abwärts und in Bezug auf die erste Mischvorrichtung in Strömungsrichtung aufwärts angeordnet ist.

28. System nach Anspruch 27, wobei die zweite Mischvorrichtung (5, 6) wenigstens einen zweiten Rohrreaktor (6) aufweist, der turbulente Mischungsbedingungen für den darin eingespeisten kombinierten Inhalt der zweiten und der dritten Behälter fördert.

29. System nach einem der Ansprüche 25 - 28, das weiterhin eine zweite Mischvorrichtung (5, 6) aufweist, die in Bezug auf den wenigstens einen ersten Behälter, der die dispersierten magnetischen Partikel (8) aufweist, und den wenigstens einen zweiten Behälter, der die eine makromolekulare Substanz oder die mehreren makromolekularen Substanzen
(1) aufweist, in Strömungsrichtung abwärts und in Bezug auf die erste Mischvorrichtung in Strömungsrichtung aufwärts angeordnet ist.

30. System nach Anspruch 29, wobei die zweite Mischvorrichtung (5, 6) wenigstens einen zweiten Rohrreaktor (6) aufweist, der turbulente Mischungsbedingungen für den darin eingespeisten kombinierten Inhalt der zweiten und der dritten Behälter fördert.

31. System nach einem der Ansprüche 25 - 30, das weiterhin wenigstens eine Recyclingleitung (23, 24, 25) zum Bereitstellen eines Durchlasses von der Auffangabteilung (16) zu einem in Bezug auf die erste Mischvorrichtung (10, 11) strömungsabwärts gelegenen Systemteils schafft.

## Revendications

1. Procédé continu d'obtention d'une préparation d'un assemblage macromoléculaire d'une ou plusieurs substances macromoléculaires comprenant au moins une substance choisie dans le groupe comprenant les protéines, les hydrates de carbone, les acides nucléiques, les analogues d'acides nucléiques et les chimères protéine-acide-nucléique, ledit procédé comprenant les étapes consistant à :
a) (i) fournir une ou plusieurs compositions liquides comprenant chacune au moins l'une desdites une ou plusieurs substances macromoléculaires, lesdites une ou plusieurs substances macromoléculaires se présentant majoritairement sous forme non assemblée, (ii) fournir une dispersion comprenant des particules magnétiques principalement non poreuses et enrobées et (iii), facultativement, fournir un ou plusieurs additifs ;
b) combiner simultanément ou une à une lesdites une ou plusieurs compositions liquides, la dispersion de particules magnétiques et lesdits un ou plusieurs additifs facultatifs de manière à fournir un écoulement continu comprenant lesdites une ou plusieurs substances macromoléculaires, les particules magnétiques et tous additifs facultatifs ;
c) faire passer ledit écoulement continu dans un premier dispositif mélangeur de manière à former des complexes magnétiques comprenant chacun une particule magnétique et une pluralité d'espèces d'assemblage macromoléculaire ;
d) faire passer l'écoulement continu dans un premier compartiment de capture d'un séparateur magnétique, de manière à capturer lesdits complexes magnétiques ; et
e) séparer lesdits complexes magnétiques dudit écoulement continu, et isoler lesdites espèces d'assemblage macromoléculaire desdites particules magnétiques de manière à obtenir la préparation d'un assemblage macromoléculaire d'une ou plusieurs substances macromoléculaires.

2. Procédé selon la revendication 1, dans lequel lesdites une ou plusieurs substances macromoléculaires sont choisies parmi les protéines.

3. Procédé selon la revendication 1, dans lequel lesdites une ou plusieurs substances macromoléculaires sont choisies parmi les acides nucléiques, les analogues d'acides nucléiques et les chimères protéine-acide-nucléique.

4. Procédé selon l'une quelconque des revendications précédentes, dans lequel le poids moléculaire de chacune desdites une ou plusieurs substances macromoléculaires est d'au moins 1 000 Da.

5. Procédé selon l'une quelconque des revendications précédentes, dans lequel une seule substance macromoléculaire est nécessaire pour former l'assemblage macromoléculaire.

6. Procédé selon la revendication 5, dans lequel la substance macromoléculaire est représentée par une protéine unique, la forme non assemblée de ladite protéine étant la forme dépliée, mal repliée ou agrégée, et l'assemblage moléculaire de ladite protéine étant la forme repliée biologiquement active.

7. Procédé selon l'une quelconque des revendications 1 à 5, dans lequel au moins deux substances macromoléculaires sont nécessaires pour former l'assemblage macromoléculaire.

8. Procédé selon la revendication 7, dans lequel au moins deux substances macromoléculaires sont représentées par une pluralité de protéines, la forme non assemblée desdites protéines correspondant aux protéines respectives sous la forme dépliée, mal repliée ou agrégée, et l'assemblage macromoléculaire desdites protéines étant une structure quaternaire biologiquement active où les protéines respectives représentent des sous-unités ou domaines.

9. Procédé selon la revendication 8, dans lequel les au moins deux substances macromoléculaires sont représentées par une pluralité d'espèces choisies parmi les acides nucléiques, les analogues d'acides nucléiques et les chimères protéine-acide-nucléique, la forme non assemblée desdites substances correspondant aux formes non hybridées desdites espèces, et l'assemblage moléculaire desdites substances étant la forme d'hybridation mutuelle desdites espèces.

10. Procédé selon l'une quelconque des revendications précédentes, dans lequel les particules magnétiques sont superparamagnétiques.

11. Procédé selon l'une quelconque des revendications précédentes, dans lequel les particules magnétiques portent un ou plusieurs types de ligands.

12. Procédé selon la revendication 11, dans lequel le(s) ligand(s) choisi(s) a(ont) une affinité pour l'une au moins desdites une ou plusieurs substances macromoléculaires.

13. Procédé selon la revendication 11, dans lequel le(s) ligand(s) choisi(s) a(ont) une affinité pour l'assemblage macromoléculaire.

14. Procédé selon la revendication 13, dans lequel le(s) ligand(s) choisi(s) n'a(ont) sensiblement aucune affinité pour les substances macromoléculaires individuelles non assemblées.

15. Procédé selon l'une quelconque des revendications précédentes, dans lequel, à l'étape (b), on combine lesdites une ou plusieurs compositions liquides et lesdits un ou plusieurs additifs facultatifs et on les fait passer dans un second dispositif mélangeur, de manière à former un pré-écoulement continu d'assemblage macromoléculaire d'une ou plusieurs substances macromoléculaires, ledit pré-écoulement étant ensuite combiné à la dispersion de particules magnétiques de manière à former l'écoulement continu mentionné à l'étape (b).

16. Procédé selon l'une quelconque des revendications 1 à 14, dans lequel, à l'étape (b), on combine l'une au moins desdites une ou plusieurs compositions liquides et la dispersion de particules magnétiques, et on les fait passer dans un second dispositif mélangeur, de manière à former un pré-écoulement continu de complexes magnétiques de substances magnétiques et d'une ou plusieurs substances macromoléculaires, ledit pré-écoulement étant ensuite combiné à n'importe laquelle desdites une ou plusieurs compositions liquides restantes et desdits un ou plusieurs additifs facultatifs de manière à former l'écoulement continu mentionné à l'étape (b).

17. Procédé selon l'une quelconque des revendications 1 à 14, dans lequel, à l'étape (b), on combine sensiblement simultanément lesdites une ou plusieurs compositions liquides, la dispersion de particules magnétiques et un ou plusieurs additifs facultatifs, de manière à former l'écoulement continu mentionné à l'étape (b).

18. Procédé selon l'une quelconque des revendications précédentes, dans lequel le premier dispositif mélangeur favorise des conditions de mélange turbulent des constituants dudit écoulement.

19. Procédé selon l'une quelconque des revendications précédentes, dans lequel le séparateur magnétique comporte au moins deux modes opératoires, le premier desdits au moins deux modes opératoires fournissant un champ magnétique au premier compartiment de capture en rendant ainsi celui-ci apte à capturer les complexes magnétiques, et le second desdits au moins deux modes opératoires fournissant un champ magnétique inapproprié au premier compartiment de capture, rendant ainsi celui-ci inapte à capturer les complexes magnétiques.

20. Procédé selon l'une quelconque des revendications précédentes, dans lequel on sépare les complexes magnétiques de l'écoulement continu en redirigeant ledit écoulement continu vers un second compartiment de capture.

21. Procédé selon la revendication 20, dans lequel le second compartiment de capture est un second compartiment de capture du séparateur magnétique mentionné à la revendication N° 1.

22. Procédé selon l'une quelconque des revendications 1 à 19, dans lequel on sépare les complexes magnétiques de l'écoulement continu en interrompant ledit écoulement.

23. Procédé selon l'une quelconque des revendications précédentes, dans lequel une fraction au moins de l'écoulement continu, après son passage dans le compartiment de capture du séparateur magnétique, est continûment renvoyée en amont de manière à intégrer l'écoulement continu de l'étape (b).

24. Procédé selon l'une quelconque des revendications précédentes, dans lequel les particules magnétiques sont renvoyées en amont et mélangées à la dispersion de particules magnétiques, ou renvoyées en amont vers n'importe lequel des mélangeurs (10, 11, 5, 6), après isolation de l'assemblage macromoléculaire.

25. Système utile à l'assemblage continu et à la capture de substances macromoléculaires, le système comprenant : une pluralité de récipients comprenant au moins un premier récipient contenant une dispersion de particules magnétiques principalement non poreuses et enrobées (8), au moins un deuxième récipient contenant une composition liquide comprenant une ou plusieurs substances macromoléculaires (1) et, facultativement, au moins un troisième récipient (2) contenant un ou plusieurs additifs ; au moins une pompe (3, 4, 7) permettant d'envoyer le contenu de chacun desdits récipients (1, 2, 8) vers un premier dispositif mélangeur (10, 11); une conduite assurant le passage dudit premier dispositif mélangeur (10, 11) au premier compartiment de capture (16) d'un séparateur magnétique (15), ledit séparateur magnétique (15) comportant au moins deux modes opératoires, le premier des au moins deux modes opératoires fournissant un champ magnétique au premier compartiment de capture (16) en rendant ainsi celui-ci apte à capturer les particules magnétiques (8), et le second des au moins deux modes opératoires fournissant un champ magnétique inapproprié au premier compartiment de capture (16), rendant ainsi celui-ci sensiblement inapte à capturer les complexes magnétiques (8).

26. Système selon la revendication 25, dans lequel le premier dispositif mélangeur (10, 11) comprend au moins un premier réacteur tubulaire (11) pour favoriser les conditions de mélange turbulent du contenu desdits récipients (1, 2, 8).

27. Système selon l'une quelconque des revendications 25 et 26, comprenant en outre un second dispositif mélangeur (5, 6) installé en aval dudit au moins un deuxième récipient contenant lesdites une ou plusieurs substances macromoléculaires (1) et dudit au moins un troisième récipient contenant un ou plusieurs additifs (2), et en amont dudit premier dispositif mélangeur.

28. Système selon la revendication 27, dans lequel ledit second dispositif mélangeur (5, 6) comprend au moins un second réacteur tubulaire (6) favorisant les conditions de mélange turbulent du contenu combiné desdits deuxième et troisième récipients qui lui est envoyé.

29. Système selon l'une quelconque des revendications 25 à 28, comprenant en outre un second dispositif mélangeur (5, 6) installé en aval dudit au moins un premier récipient contenant les particules magnétiques dispersées (8) et dudit au moins un deuxième récipient contenant lesdites une ou plusieurs substances macromoléculaires (1), et en amont dudit premier dispositif mélangeur.

30. Système selon la revendication 29, dans lequel ledit second dispositif mélangeur (5, 6) comprend au moins un second réacteur tubulaire (6) favorisant les conditions de mélange turbulent du contenu combiné desdits deuxième et troisième récipients qui lui est envoyé.

31. Système selon l'une quelconque des revendications 25 à 30, comprenant en outre au moins une conduite de recyclage (23, 24, 25) assurant un passage du compartiment de capture (16) à un élément du système situé en amont du premier dispositif mélangeur (10,11).
